# FASCICULE DE BREVET EUROPEEN

(11) **EP 3 207 920 B1**
(45) Date de publication et mention de la délivrance du brevet: **10.08.2022**
(21) Numéro de dépôt: 17158774.4
(22) Date de dépôt: 14.01.2008
(51) Int. Cl.: A61K 8/67, A61K 8/68, A61Q 1/04, A61Q 1/06, A61P 17/02, A61Q 19/00, A61P 17/16

(54) **UTILISATION D'ACTIFS PERMETTANT D'AUGMENTER LA TENEUR EN CERAMIDES DANS LES LEVRES, A TITRE D'AGENT PROTECTEUR DES LEVRES FRAGILES**
VERWENDUNG VON AKTIVSTOFFEN, DIE EINE ERHÖHUNG DES CERAMIDGEHALTS IN LIPPEN BEWIRKEN, ALS SCHUTZSUBSTANZEN FÜR SPRÖDE LIPPEN
USE OF ACTIVE AGENTS FOR INCREASING THE CERAMIDE CONTENT OF THE LIPS, AS A PROTECTION AGENT FOR FRAGILE LIPS

(30) Priorité: 12.01.2007 FR 0752658; 16.07.2007 FR 0756522
(43) Date de publication de la demande: 23.08.2017
(62) Demande divisionnaire de: 08150223.9
(73) Titulaire: L'OREAL, 75008 Paris (FR)
(72) Inventeur: BOUSSOUIRA, Boudiaf, 94400 VITRY SUR SEINE (FR); JAMMAYRAC, Odette, 94152 CHEVILLY LA RUE (FR); DE LACHARRIERE, Olivier, 75015 PARIS (FR); BURNIER, Véronique, 94152 CHEVILLY LA RUE (FR)
(74) Mandataire: Nony

(56) Documents cités:
- EP-A- 0 662 319
- EP-A- 1 600 152
- EP-A1- 0 500 437
- EP-A1- 0 647 617
- EP-A1- 0 716 849
- WO-A-01/76538
- WO-A-01/82861
- WO-A-99/09936
- WO-A-99/09937
- FR-A- 2 759 902
- FR-A- 2 835 430
- FR-A1- 2 855 048
- FR-A1- 2 855 049
- US-A1- 2003 095 936
- US-A1- 2004 092 482
- DATABASE WPI Week 199222 Thomson Scientific, London, GB; AN 1992-180100 XP002473704, & JP 4 117317 A (KANEBO LTD) 17 avril 1992 (1992-04-17)
- DATABASE WPI Week 200657 Thomson Scientific, London, GB; AN 2006-553046 XP002473705, & JP 2006 193492 A (NIPPON TENNENBUTSU KENKYUSHO KK) 27 juillet 2006 (2006-07-27)

## Description

La présente invention se rapporte au domaine de la protection préventive et/ou curative des lèvres fragiles.

Par « lèvres fragiles » dans la cadre de la présente invention, on entend désigner des lèvres susceptibles d'être affectées par un certain nombre de signes ou encore troubles allant de sensations d'inconforts aux gerçures, en passant par des sensations de picotements et de tiraillements, l'apparition de squames, de plaques sèches, de fendillements ou encore de saignements. Ces signes sont plus particulièrement localisés sur la partie des lèvres dénommée vermillon ou lèvres rouges. Les études épidémiologiques réalisées sur des centaines de femmes en Europe, en Asie et en Amérique du Nord montrent que les lèvres fragiles constituent un désordre fréquent. Ainsi, par exemple, il touche 67 % des femmes en France et 54 % au Japon. Ce désordre est ressenti comme sévère (lèvres très fragiles) par une femme sur cinq en France et une femme sur trois au Japon.

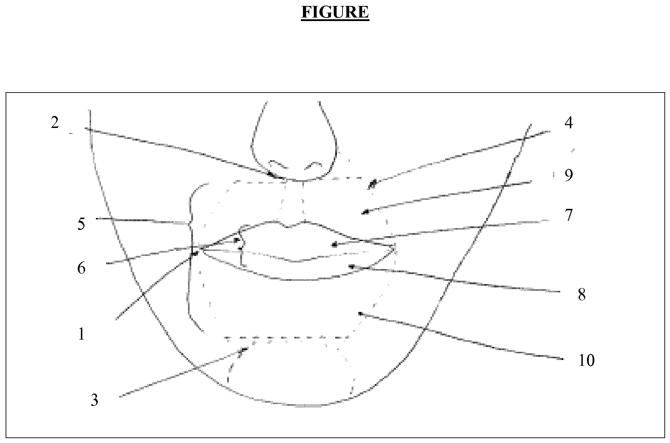

La figure annexée permet d'illustrer cette zone des vermillons et plus généralement l'anatomie de la région péri-labiale.

Le terme « lèvre » recouvre en fait une région anatomique du visage référencée 5 dans la figure annexée, délimitée en haut par le nez 2, latéralement par les sillons naso-géniens 4 et en bas par la houppe du menton 3. Elle comporte la lèvre supérieure 9 et la lèvre inférieure 10. La lèvre supérieure et la lèvre inférieure s'unissent à leurs deux extrémités latérales pour former les commissures labiales 1.

Trois parties bien différenciées sont par ailleurs à distinguer au sein de cette entité anatomique :
- une partie dont l'apparence est similaire à celle de la peau du corps. Il s'agit des « lèvres blanches »,
- une partie dont l'apparence est très différente de la peau du corps, il s'agit des « lèvres rouges », aussi appelée vermillon,
- une partie endobuccale, de type muqueuse.

Les lèvres blanches sont constituées par un tissu cutané conventionnel, orthokératinisé, revêtu d'un épiderme et d'un derme pourvu de follicules pilo-sébacées et de glandes sudorales eccrines.

La peau conventionnelle a pour fonction principale d'établir une barrière de protection contre les agressions de l'environnement tout en permettant certains échanges entre le milieu intérieur et le milieu extérieur. Elle est constituée de l'intérieur vers l'extérieur, par l'hypoderme, le derme et l'épiderme. L'épiderme est la couche la plus superficielle de la peau, composée majoritairement par les kératinocytes (90%). La différenciation des kératinocytes s'accompagne d'un ensemble de transformations morphologiques et biochimiques qui aboutit à la formation de cellules matures, les cornéocytes. Les cornéocytes sont des cellules anucléées et aplaties. Elles se détachent à la surface de la peau selon un processus de desquamation. Cette desquamation lorsqu'elle est physiologique est inapparente et ne donne pas lieu à la présence de squames visibles à la surface de la peau. Au cours de leur maturation cornée, les kératinocytes migrent de la profondeur vers la surface de l'épiderme, et forment quatre couches épidermiques : la couche basale (stratum basale), la couche épineuse (stratum spinosum), la couche granuleuse (stratum granulosum), et la couche cornée (stratum corneum), qui est la couche la plus externe, composée par des cornéocytes matures. Cette couche construit la première ligne de défense de la barrière épidermique.

Le vermillon 6 présente quant à lui une structure très particulière, aucunement assimilable avec la structure de la peau conventionnelle, à plusieurs titres.

La zone vermillon ou lèvres rouges (inférieure 8 ou supérieure 7) est une structure unique aux homos sapiens. Il s'agit d'une zone intermédiaire entre la peau orthokératinisée et la muqueuse. Elle est constituée d'un épithélium parakératinisé, c'est-à-dire présentant une kératinisation incomplète des cornéocytes, dont certains contiennent encore des éléments nucléés. Le derme présente de très nombreuses et importantes papilles dermiques (dessinant une hyperpapillomatose physiologique sur les coupes histologiques).

De plus, l'épithélium du vermillon est plus épais par rapport à la peau conventionnelle, et notamment par rapport à la peau des lèvres blanches. Par contre, le stratum corneum (SC) recouvrant le vermillon est extrêmement fin. Les kératinocytes du vermillon ont un taux de renouvellement ou « turnover » plus rapide que dans la peau conventionnelle. Cette rapidité induit une différentiation incomplète des cornéocytes (kératinisation incomplète), donc un SC immature contenant des cellules nucléées (Hikima R.et al. Etude fondamentale des lèvres (II) : Relief labial et gercement des lèvres. JOURNAL OF JAPANESE COSMETIC SCIENCE SOCIETY 20[4], 265-69. 1996). Le taux de ces cellules nucléées semble corrélé positivement avec le degré de gercement des lèvres.

Le vermillon est richement vascularisé et repose quasi-directement - sans véritable assise hypodermique - sur le muscle orbiculaire des lèvres, l'ensemble donnant cette couleur rouge vermillon à cette partie des lèvres. Les vermillons, à la différence des autres zones du revêtement cutané du corps, ne contiennent pas de follicule pileux, pas de glandes sudorales et peu ou pas de glandes sébacées. Pour ces dernières, seul 50 % des individus ont quelques glandes sébacées, qui apparaissent après l'adolescence.

En conséquence, la physiologie de cette zone très particulière de la peau est très spécifique et très mal connue. On ne peut pas, compte tenu de ces nombreuses particularités (différenciation parakératosique, assise musculaire, absence d'annexes épidermiques), transposer les connaissances établies pour le reste du revêtement cutané à l'épiderme du vermillon.

Enfin, la muqueuse est une zone intérieure revêtue d'un épithélium épais, pavimenteux non kératinisé, dépourvue de SC, et dotée d'une grande perméabilité. Cette zone est maintenue continuellement humide par la salive.

Dans la suite de la présente description, pour des raisons de simplicité, et bien que la zone des vermillons soit la zone visée par la présente description, on utilisera le terme « lèvres ». Autrement dit, dans la suite de la description, sauf indication contraire, le terme « lèvres » désigne les vermillons.

Afin de bien appréhender la présente invention, il faut ainsi noter la spécificité des vermillons non seulement en terme histologique comme évoqué précédemment, en particulier vis-à-vis de la peau, mais encore en terme d'affections ou signes de gêne, voire de pathologies qui peuvent leur être associées.

En réalité, il a pu être constaté que certaines femmes se plaignent de lèvres fragiles toute l'année. Elles sont alors considérées comme ayant des lèvres « très fragiles ». D'autres se plaignent de lèvres fragiles seulement au cours de certaines périodes de l'année, par l'exemple l'hiver. Elles sont alors considérées comme ayant des lèvres « fragiles ».

Sauf indication contraire dans la description qui va suivre, le terme « lèvres fragiles » employé recouvre les lèvres « fragiles » et les lèvres « très fragiles » définies précédemment.

En tout état de cause, compte tenu de sa localisation à la frontière de la sphère digestive et cutanée, des nombreux contacts avec les aliments et la salive et de sa structure particulière avec un *stratum corneum* fin et immature, le vermillon représente chez beaucoup de personnes une zone de fragilité reconnue par ces dernières.

Les signes associés aux lèvres fragiles sont détaillés ci-après. Ils peuvent exister séparément les uns des autres ou être associés.

Tous ces signes peuvent être désagréables, gênants et inconfortables lors des mouvements des lèvres au cours de la parole ou de l'alimentation. De plus, ils peuvent empêcher l'utilisation de rouges à lèvres. Dans les cas les plus sévères où la femme présente des fendillements ou des saignements, ces derniers signes peuvent être douloureux : ils méritent donc que l'on y prête attention en vue de mettre au point des méthodes cosmétologiques de prévention et/ou de traitement appropriées.

Bien que l'ensemble de ces signes soit fréquemment observé, l'amplification des phénomènes déclencheurs est loin d'être maîtrisée. On connaît notamment l'influence de l'âge sur la survenue de ces signes. (Arais S., et al. Etat de surface normal des lèvres (I) : le gercement des lèvres. Study on lip surface : Characteristics of chapped lip. JOURNAL OF JAPANESE COSMETIC SCIENCE SOCIETY 13[2], 64-68. 1989).

Par ailleurs, les lèvres sont exposées en permanence à l'environnement extérieur, subissant directement les agressions environnementales. Par exemple, le froid et le vent pourraient être des facteurs desséchants pour les lèvres et contribuer à l'apparition de lèvres fragiles. La saison d'hiver a été montrée comme étant une période plus propice au gercement sur les lèvres. Les UV sont aussi susceptibles d'être une cause des lèvres fragiles. Dans la société actuelle, l'utilisation répandue de l'air conditionné pourrait également jouer un rôle dans la survenue des lèvres fragiles.

Enfin, les lèvres sont directement en contact avec différentes stimulations de nature physico-chimique. Par exemple la prise de repas, les rouges à lèvres, le tabagisme, la langue, etc, font partie des facteurs provoquant des lèvres fragiles.

Ainsi, lorsqu'il est classiquement question de protéger les lèvres, on vise généralement à pallier uniquement le problème des lèvres desséchées et de la formation de gerçures ou de crevasses sur les lèvres.

Toutefois, comme décrit précédemment, le problème des lèvres fragiles relève d'un concept plus complexe. Ainsi, les lèvres fragiles occasionnent non seulement des dommages mécaniques et biochimiques de la barrière épidermique et des anomalies de desquamation, mais aussi jouent sur l'aspect neurosensoriel des lèvres.

Ainsi, toutes les personnes concernées par les lèvres fragiles au sens de la présente invention ne peuvent pas être satisfaites par les produits couramment employés, à savoir notamment les baumes à lèvre en stick visant principalement l'hydratation des lèvres qui permet de calmer les effets immédiats du dessèchement ou de gerçures. On peut aussi reprocher à ces baumes à lèvres de l'art antérieur de ne même par faire preuve de rapidité d'action, voire même de ne pas apporter de réelle solution au problème lui-même.

Typiquement, on connaît l'utilisation de baumes à lèvres contenant des corps gras, notamment des huiles ou cires, par exemple d'origine végétale, de la vaseline ou encore de la lanoline.

Ces substances ont pour but principal de freiner la perte en eau à partir des lèvres.

Ces baumes à lèvres peuvent en outre présenter l'inconvénient de conférer un aspect peu esthétique du fait de propriétés cosmétiques d'application médiocre en raison du gras et de la lourdeur des substances hydratantes précitées.

Il est par ailleurs connu du document EP 0 940 137 d'utiliser de l'acide acexamique à titre d'agent permettant de traiter et d'empêcher le dessèchement et la formation de gerçures et/ou de crevasses des lèvres.

Les céramides constituent environ 47 % du poids total des lipides du SC de la peau et sont également présents en grande quantité dans les lèvres.

Ils sont composés d'une base sphingoïde qui peut être de quatre types : sphinganine (encore appelée dihydrosphingosine), 4-sphingénine (encore appelée sphingosine), phytosphingosine (encore appelée 4-hydroxy-sphinganine) et 6-hydroxy-4-sphingénine (encore appelée 6-hydroxy-sphingénine), et d'un acide gras qui peut être saturé, α-hydroxylé ou ω-estérifié. Les différentes combinaisons possibles entre bases et acides gras conduisent à une dizaine de céramides répertoriés par Robson,K.J.; Stewart,M.E.; Michelsen,S.; Lazo,N.D.; Downing,D.T., 6-hydroxy-4-sphingenine in human epidermal ceramides, dans J. Lipid Res. 1994 35 : 2060-2068; et Chopart M., Castiel-Higounenc I., Arbey E., Guey C., Gaetani Q., Schmidt R., The Normal Human stratum corneum: α new ceramide profile, Perspectives in Percutaneous Penetration, 8th International Conference, Antibes Juan-Les Pins - France, April 2-6,2002.

Un certain nombre de céramides naturels ou de synthèse et de leurs précurseurs a été proposé comme actif dans des compositions destinées à traiter des désordres cutanés tels que les dommages causés par les UV ou les déficiences en hydratation.

Ainsi le document FR 2 855 049 enseigne l'utilisation, pour renforcer la fonction barrière de la peau et notamment pour lutter contre les désordres cutanés tels que les peaux sèches, rugueuses, abîmées, voire âgées, d'une composition comprenant une substance capable d'activer spécifiquement la synthèse *in vivo* de céramides à base 6-hydroxy-4-sphingénine, à l'image par exemple de l'acide ascorbique (vitamine C) et de ses analogues, en association avec un précurseur de céramide à base sphingénine ou un de ses dérivés.

Pour sa part, le document FR 2 855 048 enseigne l'utilisation de l'association d'au moins un précurseur de céramide à base phytosphingosine et d'une substance activatrice de la voie des 4-hydroxylases également pour renforcer la fonction barrière de la peau et en particulier pour pallier aux désordres cutanés de type peaux sèches, rugueuses, abîmées, et/ou atopiques, voire âgées ou sensibles.

Le document FR 2 835 430 se rapporte à des compositions utiles pour le soin des peaux sensibles, comprenant au moins un homopolymère ou copolymère de méth(acrylamide) et une suspension aqueuse de nanocapsules au moins partiellement enrobées de lécithine.

Les documents EP 0 500 437 et EP 0 647 617 décrivent des céramides de synthèse pour le traitement et le soin de la peau et des cheveux.

Quant au document EP 0 716 849, il décrit une composition contenant un mélange de céramides, destiné en particulier à hydrater la peau.

Le document FR 2 759 902 divulgue pour sa part des bâtons de rouge à lèvres contenant, entre autres, des céramides et ayant également des propriétés hydratantes tout en ayant une bonne tenue au maquillage.

WO 01/75538 divulgue également l'utilisation d'une composition contenant des céramides, pour le traitement des lèvres soumises à un stress xérique.

Enfin, le document EP 1 272 148 décrit l'utilisation d'un mélange de céramides, d'acides gras essentiels et d'acides gras non essentiels dans des rapports molaires définis, pour la fabrication d'un médicament pour la prévention et la réparation du stress xérique des lèvres d'un sujet humain par application auxdites lèvres d'une quantité de ladite composition permettant un maintien de la fonction barrière.

Les inventeurs ont constaté, de façon surprenante, que l'apparition des lèvres fragiles au sens de la présente invention est liée à un déficit en céramides à base 6-hydroxy-4-sphingénine.

Parmi les céramides à base 6-hydroxy-4-sphingénine, on peut notamment citer le céramide de type IV, le céramide de type VII, le céramide de type 5,5 et le céramide STAR.

Par céramide STAR, on entend désigner au sens de l'invention les composés de structure : 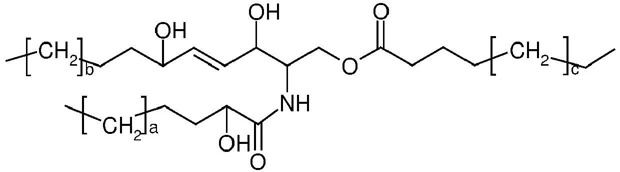 dans laquelle a, b et c sont des entiers variant de 3 à 30, de préférence de 8 à 24, et en particulier dans laquelle a varie de 15 à 20, b varie de 7 à 18 et c varie de 10 à 18.

Les inventeurs ont ainsi trouvé qu'il était possible de combler ce déficit en céramides à base 6-hydroxy-4-sphingénine dans les lèvres par la mise en œuvre d'un précurseur des céramides à base 6-hydroxy-4-sphingénine ou de l'acide ascorbique et ses analogues.

Ainsi, un précurseur des céramides à base 6-hydroxy-4-sphingénine ou l'acide ascorbique et ses analogues jouent le rôle d'actifs permettant d'augmenter la teneur en céramides dans les lèvres, en particulier les lèvres fragiles

En effet, ils ont trouvé qu'un précurseur de céramides à base 6-hydroxy-4-sphingénine est susceptible de se transformer en céramides correspondants, par exemple par hydroxylation de sa position 6 par des enzymes telles que les 6-hydroxylases et permet ainsi la restauration de la teneur en céramides dans les lèvres.

En outre, les inventeurs ont constaté, de façon inattendue, que pour sa part, l'acide ascorbique et ses analogues permettaint notamment d'accéder plus rapidement à une augmentation de la teneur en céramides à base 6-hydroxy-4-sphingénine dans les lèvres, notamment en augmentant le taux de conversion de précurseurs de céramides à base 6-hydroxy-4-sphingénine, présents naturellement dans les lèvres en céramides à base 6-hydroxy-4-sphingénine. En effet, l'acide ascorbique et ses analogues permettent d'activer la voie des 6-hydroxylases, enzymes impliquées dans la transformation des précurseurs de céramides à base 6-hydroxy-4-sphingénine en céramides à base 6-hydroxy-4-sphingénine, par hydrolyse en position 6 de la base constitutive d'au moins un précurseur précité.

L'invention concerne donc selon un premier objet, une composition pour application labiale comprenant, dans un milieu physiologiquement acceptable, au moins la N-oléoyldihydrosphingosine et la α-hydroxypalmitoyldihydrosphingosine.

Le présent texte décrit, outre l'invention revendiquée, l'utilisation cosmétique d'un actif permettant d'augmenter la teneur en céramides dans les lèvres, notamment en céramide à base 6-hydroxy-4-shingénine, choisi parmi un précurseur de céramide à base 6-hydroxy-4-sphingénine, l'acide ascorbique ou au moins l'un de ses analogues à titre d'agent protecteur des lèvres fragiles, en particulier la zone des vermillons.

Est également décrite dans le présent texte, l'utilisation cosmétique d'un précurseur de céramide à base 6-hydroxy-4-sphingénine, à titre d'agent protecteur des lèvres fragiles, et en particulier la zone des vermillons.

Plus particulièrement est décrite l'utilisation cosmétique d'un composé précurseur de céramide à base 6-hydroxy-4-sphingénine, de formule (I) suivante :

R₁-CHOH-CH(NH-COR₂)(CH₂OH) (I)

dans laquelle :
- R₁ représente un radical alkyle ou alcényle en C₃ à C₃₀, en particulier en C₇ à C₂₅, et de préférence, en C₁₁ à C₂₁, et
- R₂ représente un atome d'hydrogène ou un radical hydrocarboné en C₃ à C₃₀, en particulier en C₅ à C₂₅, et de préférence en C₈ à C₂₄, linéaire, éventuellement hydroxylé et le groupe hydroxyle étant en position alpha du carbonyle, et pouvant comporter une ou plusieurs insaturations éthyléniques, notamment une ou deux insaturations éthyléniques ou de l'un de ses dérivés, à titre d'agent protecteur des lèvres fragiles, en particulier la zone des vermillons.

Plus particulièrement, est décrite l'utilisation cosmétique d'un céramide V ou d'un dérivé de céramide V tel que décrit ci-après à titre d'agent protecteur des lèvres fragiles, en particulier la zone des vermillons.

Le présent texte décrit également, outre l'invention revendiquée, l'utilisation cosmétique de l'acide ascorbique ou d'au moins l'un de ses analogues, à titre d'agent protecteur des lèvres fragiles et en particulier la zone des vermillons.

Par « analogue » de l'acide ascorbique, on entend désigner au sens de l'invention, les sels, les esters, les sucres et les dérivés de l'acide ascorbique susceptibles de manifester la même activité que l'acide ascorbique vis-à-vis de la protection des lèvres fragiles, et en particulier vis-à-vis de la prévention et/ou du traitement des signes cliniques associés aux lèvres fragiles.

Les signes associés aux lèvres fragiles sont détaillés ci-après. Ils peuvent exister séparément les uns des autres ou être associés.
- la sensation d'inconfort et/ou de picotements : il s'agit d'un état sur lequel il est souhaitable d'apporter une réponse, de traitement ou de soin, qui permette de mieux supporter une gêne.
- la sensation de tiraillements : il s'agit d'une sensation de tension gênante sur les lèvres.
- la présence de squames : il s'agit de petites lamelles de stratum corneum qui se détachent de façon visible de l'épiderme.
- les plaques sèches : il s'agit de petites zones du stratum corneum épaissies, durcies, qui se détachent en plaques.
- les fendillements : il s'agit de petites plaies, apparaissant spontanément, au sein de la partie superficielle de l'épiderme, qui ont un aspect de coupures superficielles perpendiculaires à la surface des lèvres ; elles n'entraînent pas forcément de saignement.
- les fissures : il s'agit de fendillements plus importants dont la profondeur atteint l'épiderme profond et le derme.
- les saignements : il s'agit de suintement de sang par les fendillements ou fissures des lèvres.

L'expression « lèvres fragiles », en particulier la zone des vermillons regroupe au sens de l'invention les lèvres, en particulier la zone des vermillons affectées par au moins l'un de ces signes associés aux lèvres fragiles en particulier de la zone des vermillons. On peut schématiquement décrire trois stades cliniques de sévérité croissante :
- stade 1 : inconforts et/ou picotements et/ou tiraillements ;
- stade 2 : aux signes du stade 1 s'associent des squames et/ou des plaques sèches ;
- stade 3 : aux signes des stades 1 et 2 s'associent des fendillements et/ou des fissures et/ou des saignements.

Les précurseurs de céramide à base 6-hydroxy-4-sphingénine ou l'acide ascorbique et ses analogues, utilisés à titre d'agent protecteur des lèvres fragiles, en particulier la zone des vermillons, permettent précisément de prévenir la manifestation au niveau des lèvres fragiles des signes cliniques précités et/ou de traiter des lèvres manifestant au moins l'un de ces signes, et donc d'avoir un effet réparateur.

Ainsi, un précurseur de céramide à base 6-hydroxy-4-sphingénine ou l'acide ascorbique ou au moins l'un de ses analogues permet notamment de prévenir et/ou de traiter les signes associés aux lèvres fragiles, en particulier la zone des vermillons, de type sensations d'inconfort et/ou picotements et/ou tiraillements

Un précurseur de céramide à base 6-hydroxy-4-sphingénine ou l'acide ascorbique ou au moins l'un de ses analogues permettent, par ailleurs, de prévenir et/ou de traiter les signes associés aux lèvres fragiles, en particulier la zone des vermillons, se manifestant par la présence de squames et/ou de plaques sèches.

Enfin, un précurseur de céramide à base 6-hydroxy-4-sphingénine ou l'acide ascorbique ou au moins l'un de ses analogues permettent également de prévenir et/ou de traiter les signes associés aux lèvres fragiles, en particulier la zone des vermillons, de type fendillements et/ou fissures et/ou saignements.

Le présent texte décrit alors, outre l'invention revendiquée, l'utilisation cosmétique d'un composé précurseur de céramide à base 6-hydroxy-4-sphingénine ou de l'acide ascorbique ou d'au moins l'un de ses analogues à titre d'agent apte à prévenir et/ou traiter (i) les sensations d'inconfort et/ou de picotements et/ou de tiraillements, (ii) la présence de squames et/ou de plaques sèches, et/ou (iii) les fendillements et/ou les fissures et/ou les saignements, notamment chez des sujets ayant des lèvres fragiles.

L'invention concerne ainsi, selon un deuxième objet, une composition selon l'invention pour son utilisation dans le traitement des signes localisés sur les vermillons des lèvres associés aux lèvres fragiles choisis parmi :
- la sensation d'inconfort et/ou de picotements ;
- la sensation de tiraillements ;
- la présence de squames ;
- les plaques sèches ;
- les fendillements ;
- les fissures ; et/ou
- les saignements,
la N-oléoyldihydrosphingosine et la α-hydroxypalmitoyldihydrosphingosine étant de conformation érythro (2S, 3R).

Le présent texte décrit donc l'utilisation cosmétique d'un composé précurseur de céramide à base 6-hydroxy-4-sphingénine ou de l'acide ascorbique ou d'au moins l'un de ses analogues à titre d'agent apte à prévenir et/ou traiter (i) les sensations d'inconfort et/ou de picotements et/ou de tiraillements et (ii) la présence de squames et/ou de plaques sèches, en particulier chez des sujets ayant des lèvres fragiles.

Il est également décrit un procédé de traitement cosmétique de sujets ayant des lèvres fragiles, en particulier la zone des vermillons, comprenant l'application sur les lèvres desdits sujets d'une composition comprenant au moins un précurseur de céramide à base 6-hydroxy-4-sphingénine ou au moins de l'acide ascorbique ou au moins l'un de ses analogues selon l'invention.

Plus précisément, il est décrit un procédé de traitement cosmétique de sujets dont les lèvres sont affectées par des sensations d'inconfort et/ou de tiraillements et/ou de picotements, ou sont prédisposées à manifester ces sensations, comprenant l'application sur les lèvres desdits sujets d'une composition comprenant au moins un précurseur de céramide à base 6-hydroxy-4-sphingénine ou au moins de l'acide ascorbique ou au moins l'un de ses analogues.

Il est également décrit un procédé de traitement cosmétique de sujets dont les lèvres sont affectées par des squames et/ou des plaques sèches, associés ou non à une sensation d'inconfort et/ou des tiraillements et/ou des picotements, ou sont prédisposées à manifester ces troubles, comprenant l'application sur les lèvres desdits sujets d'une composition comprenant au moins un précurseur de céramide à base 6-hydroxy-4-sphingénine ou au moins l'acide ascorbique ou au moins l'un de ses analogues.

Il est également décrit un procédé de traitement cosmétique de sujets dont les lèvres sont affectées par des fendillements et/ou des fissures et/ou des saignements, associés ou non à des squames et/ou des plaques sèches, et/ou une sensation d'inconfort et/ou tiraillements et/ou des picotements ou sont prédisposées à manifester ces troubles, comprenant l'application sur les lèvres desdits sujets d'une composition comprenant au moins un précurseur de céramide à base 6-hydroxy-4-sphingénine ou au moins l'acide ascorbique ou au moins l'un de ses analogues.

Le présent texte décrit en outre l'utilisation d'un précurseur de céramide à base 6-hydroxy-4-sphingénine ou de l'acide ascorbique ou d'au moins l'un de ses analogues pour la préparation d'une composition, notamment labiale, dédiée à la protection des lèvres fragiles et en particulier la zone des vermillons, et notamment destinée au traitement et/ou à la prévention des signes cliniques associés aux lèvres fragiles au sens de l'invention.

Le présent texte décrit également un précurseur de céramide à base 6-hydroxy-4-sphingénine ou l'acide ascorbique ou au moins l'un de ses analogues pour leur utilisation comme agent protecteur des lèvres fragiles et en particulier la zone des vermillons, pour le traitement et/ou à la prévention des signes cliniques associés aux lèvres fragiles au sens de l'invention.

Le présent texte décrit également l'utilisation cosmétique d'un précurseur de céramide à base 6-hydroxy-4-sphingénine ou de l'acide ascorbique ou d'au moins l'un de ses analogues pour pallier au déficit de céramides dans les lèvres, en particulier de céramides à base 6-hydroxy-4sphingénine, chez des sujets ayant les lèvres fragiles, et en particulier pour rétablir une teneur en céramides physiologiquement acceptable, notamment en céramides à base 6-hydroxy-4sphingénine dans les lèvres.

Le présent texte décrit également des compositions cosmétiques et/ou dermatologiques, dédiées à une application labiale, comprenant dans un milieu physiologiquement acceptable un précurseur de céramide à base 6-hydroxy-4-sphingénine ou de l'acide ascorbique ou au moins l'un de ses analogues à titre d'actif destiné à protéger les lèvres fragiles et notamment la zone des vermillons, et en particulier pour prévenir et/ou traiter les signes cliniques précités.

Au sens du présent texte, le terme « prévenir » entend le fait de diminuer le risque de survenu d'un phénomène.

De façon générale, à titre de précurseur de céramide à base 6-hydroxy-4-sphingénine, on peut notamment citer les céramides V et leurs dérivés, tels que définis ci-après.

### PRECURSEUR DE CERAMIDE A BASE 6-HYDROXY-4-SPHINGENINE

On entend désigner par :
- "céramides cibles", les céramides ou dérivés de céramide à base 6-hydroxy-4-sphingénine,
- "dérivés de céramide", les glycosylcéramides ou cérébrosides (soit l'association d'un céramide avec un sucre) ou les sphingomyélines (principalement céramide phosphorylcholine ou céramide phosphoryl éthanolamine), voir la forme hydrolysée du céramide c'est à dire la base sphingoïde seule ou phosphorylée, et par
- "précurseurs de céramide à base 6-hydroxy-4-sphingénine", ou encore "précurseurs de céramide cible", ou encore de manière plus simplifiée "précurseurs de céramide" des composés de type céramide ou dérivé de céramide capables d'être transformés en céramides ou dérivés de céramide à base 6-hydroxy-4-sphingénine, encore désigné sous le terme "céramide cible".

Un précurseur de céramide cible désigne donc un céramide susceptible de se transformer en un céramide cible par exemple par hydroxylation de sa position 6. Une telle hydroxylation peut par exemple être réalisée par les 6-hydroxylases, qui sont des enzymes qui assurent la synthèse *in vivo* des céramides ou dérivés à base 6-hydroxy-4-sphingénine par hydroxylation en position 6 de la base sphingoïde de précurseurs de céramide cible.

Le précurseur de céramide ou de dérivé de céramide à base 6-hydroxy-4-sphingénine peut être un céramide ou un dérivé de céramide à base sphingosine.

Ladite base sphingosine peut être alors avantageusement de conformation érythro (2S, 3R).

Le précurseur de céramide ou de dérivé de céramide à base 6-hydroxy-4-sphingénine peut alternativement être un céramide ou un dérivé de céramide à base sphinganine.

De façon générale, il peut s'agir d'un composé de formule (I) :

R₁-CHOH-CH(NH-COR₂)(CH₂-OH) (I)

telle que définie précédemment.

Il peut notamment s'agir d'un composé de formule (I) dans laquelle R₁ représente un radical alkyle ou alcényle en C₁₄ à C₁₈, notamment en C₁₅.

Il peut également s'agir d'un composé de formule (I) dans laquelle R₂ représente un radical hydrocarboné en C₁₁ à C₁₉, de préférence en C₁₅ à C₁₉.

A titre de composé de formule (I) utilisable selon l'invention, on peut notamment citer la N-oléoyldihydrosphingosine (ou 2-oléoylamino-1,3-octadécanediol), et les céramides V.

Par céramide V, on entend désigner au sens de l'invention toute 4-sphingénine ou sphinganine acylée par un acide gras α-hydroxylé ayant de 4 à 31 atomes de carbone, notamment de 6 à 26 atomes de carbone, par exemple de 9 à 25 atomes de carbone, de préférence de 12 à 20 atomes de carbone, voire de 16 à 20 atomes de carbone.

Les céramides V peuvent ainsi être choisis parmi les composés de formule (IV) et (V) suivantes: 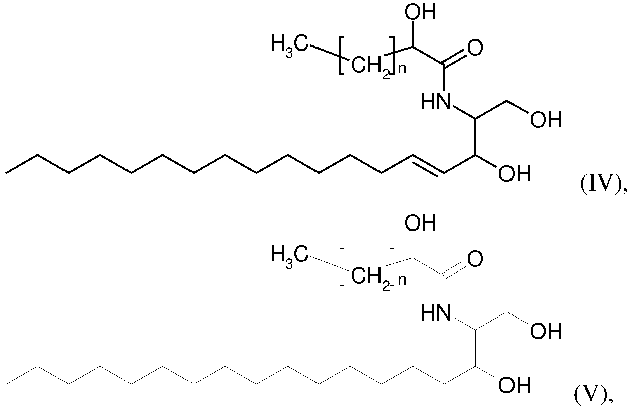 dans lesquelles n varie de 1 à 28, notamment de 3 à 23, par exemple de 6 à 22, de préférence de 9 à 17, voire de 13 à 17.

A titre de céramide V, on peut également citer la sphingosine acylée par l'acide 2-hydroxystéarique, connue aussi sous le nom 2-(2'-hydroxystéaryl)amino octadécane-1,3-diol, ou encore la α-hydroxypalmitoyldihydrosphingosine.

L'invention concerne selon un de ses aspects une composition pour application labiale comprenant, dans un milieu physiologiquement acceptable, au moins deux composés de formule (I) telle que définie précédemment.

Il s'agit d'une composition pour application labiale comprenant au moins la N-oléoyldihydrosphingosine et la α-hydroxypalmitoyldihydrosphingosine .

Selon un autre aspect, les utilisations et compositions selon l'invention peuvent mettre en œuvre au moins deux précurseurs de céramide au sens de la présente invention, en tant que seuls actifs cosmétiques et/ou dermatologiques ou bien être associé(s) à un ou plusieurs autres actifs, tel que défini dans ce qui suit.

Dans le cadre de la présente invention, la N-oléoyldihydrosphingosine et la α-hydroxypalmitoyldihydrosphingosine peuvent être mis en œuvre dans une composition cosmétique ou dermatologique et notamment dans une composition pour application labiale.

La N-oléoyldihydrosphingosine et la α-hydroxypalmitoyldihydrosphingosine peuvent être présentes dans une composition pour application labiale conforme à la présente invention dans une teneur allant de 0,0001 à 10 % en poids, par exemple de 0,001 à 5 %, et de préférence de 0,05 à 2% en poids par rapport au poids total de la composition.

Comme indiqué précédemment, selon un mode de réalisation de l'invention, la N-oléoyldihydrosphingosine et la α-hydroxypalmitoyldihydrosphingosine, peuvent être les seuls actifs utiles à titre d'agents protecteurs des lèvres fragiles.

Selon un autre mode de réalisation, elles peuvent être les seuls actifs cosmétiques et/ou dermatologiques de la composition pour application labiale. Dans ce cas, les compositions selon l'invention sont notamment exemptes d'autres actifs, et en particulier par exemple d'acide ascorbique ou de l'un de ses analogues. De telles compositions font partie de la présente invention.

Comme indiqué précédemment, selon un autre mode de réalisation, la N-oléoyldihydrosphingosine et la α-hydroxypalmitoyldihydrosphingosine peuvent être associées dans les compositions conformes à l'invention à d'autres actifs susceptibles de participer à la manifestation de l'effet recherché ou de procurer des effets complémentaires ou voire de tout autre effet pour autant qu'il ne gêne pas la manifestation de l'effet recherché, tel que défini ci-après. De telles compositions font partie de la présente invention.

Le présent texte décrit également un procédé cosmétique de soin ou de traitement non thérapeutique des lèvres des êtres humains, comprenant l'application sur les lèvres d'une composition telle que définie selon la présente invention, comprenant au moins la N-oléoyldihydrosphingosine et la α-hydroxypalmitoyldihydrosphingosine.

Comme indiqué précédemment, l'acide ascorbique et ses analogues permettent également d'augmenter la teneur en céramides dans les lèvres, notamment chez des sujets ayant les lèvres fragiles.

L'acide ascorbiques et ses analogues sont définis en détail dans ce qui suit.

### ACIDE ASCORBIQUE (vitamine C) ET SES ANALOGUES

Comme indiqué précédemment, l'acide ascorbique et ses analogues permettent d'activer la voie des 6-hydroxylases et ainsi d'accéder à une augmentation de la teneur en céramides dans les lèvres.

Appliquée au contact des lèvres, l'acide ascorbique et ses analogues vont permettre de transformer de manière spécifique les précurseurs de céramide cible présents naturellement dans les lèvres. Afin de déterminer si une substance, telle que l'acide ascorbique ou ses analogues, est activatrice de la voie des 6-hydroxylase selon l'invention on peut mettre en oeuvre toute méthode analytique permettant de quantifier le contenu des lèvres en sphingolipides, avantageusement en céramides ou dérivés de céramides, à base 6-hydroxy-4-sphingénine et mesurer si ce contenu est augmenté suite à l'application labiale de ladite substance.

De préférence, l'acide ascorbique peut être sous forme D ou L, avantageusement sous forme L, et ses analogues choisis parmi ses sels, de préférence l'ascorbate de sodium, l'ascorbylphosphate de magnésium ou de sodium, ses esters, de préférence ses esters acétique, propionique ou palmitique, ou ses sucres, de préférence l'acide ascorbique glycosylé, ainsi que les dérivés de formule (II) : 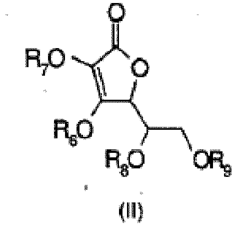 dans laquelle :
R₆ représente un atome d'hydrogène, une chaîne hydrocarbonée ayant de 15 à 20 atomes de carbone, un reste de sucre, un groupe carbonyle, un groupe alkyloxycarbonyle ou un groupe carbamoyle, ou bien R₆O représente une fonction sulfate ou une fonction phosphate,
R₇ représente un atome d'hydrogène, un reste de sucre ou un groupe -COR₁₀ où R₁₀ est choisi parmi :
   (a) un radical hydrocarboné en C₁C₂₀, saturé ou insaturé, linéaire, cyclique ou ramifié, éventuellement hydroxylé,
   (b) un radical aryle éventuellement hydroxylé,
   (c) un radical aralkyle de formule (III) : 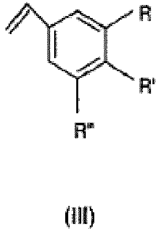
   où R, R' et R", identiques ou différents, représentent un atome d'hydrogène, un radical hydroxy, alkoxy, fluoroalkoxy ou alkylcarbonyloxy, et
R₈, R₉, identiques ou différents, représentent un atome d'hydrogène ou un groupe -COR₁₀ tel que défini précédemment ou encore, pris ensemble, forment un radical isopropylidène, ou un sel de ce dérivé.

Par "reste de sucre", on entend de préférence un reste de glucose, de galactose, de mannose, de fructose ou de N-acétylglucosamine.

Par "radical hydrocarboné saturé, linéaire ou ramifié, ayant de 1 à 20 atomes de carbone", on entend par exemple un radical choisi dans le groupe constitué par les radicaux méthyle, éthyle, propyle, iso-propyle, butyle, iso-butyle, tert-butyle, hexyle, heptyle, 2-éthyl-hexyle, octyle, nonyle, iso-nonyle, décyle, undécyle, dodécyle, pentadécyle, hexadécyle et octadécyle, et de préférence le radical undécyle.

En outre, par "radical hydrocarboné insaturé, linéaire ou ramifié, ayant de 1 à 20 atomes de carbone", on entend plus particulièrement un radical choisi dans le groupe constitué par les radicaux contenant de 2 à 5 atomes de carbone et présentant une ou plusieurs insaturations éthyléniques, en particulier le radical allyle.

Le radical aryle, substitué ou non, préféré est le radical benzyle.

Des exemples de radicaux aralkyle répondant à la formule (III) ci-dessus sont ceux dans lesquels le groupe aryle est substitué par : un ou plusieurs radicaux hydroxy ; alkoxy tels qu'un radical choisi dans le groupe constitué par les radicaux méthoxy, éthoxy et butoxy, et de préférence le radical méthoxy ; fluoroalkoxy tels que le radical trifluorométhoxy ; et alkylcarbonyloxy tels qu'un radical choisi dans le groupe constitué par les radicaux acétoxy, propionyloxy et butyryloxy, et de préférence acétoxy.

Les analogues de l'acide ascorbique convenables selon la présente invention peuvent être choisis parmi le palmitate d'ascorbyle, l'ascorbyl-2-phosphate de magnésium, l'acide glucopyranosyl-L-ascorbique, l'ascorbate de sodium, le L-ascorbyl-2-phosphate de sodium, le tetra-iso-palmitate d'ascorbyle et l'éther cétylique de vitamine C (ou vitamine C-3-O-hexadécane), et de préférence parmi le palmitate d'ascorbyle et l'éther cétylique de vitamine C.

L'éther cétylique de vitamine C est notamment disponible dans le commerce auprès de la société NIKKOL.

Selon un autre aspect, les utilisations et compositions selon l'invention peuvent mettre en œuvre l'acide ascorbique ou l'un de ses analogues associé(s) à un ou plusieurs autres actifs, tels que définis dans ce qui suit.

Comme précisé précédemment, l'acide ascorbique et/ou au moins l'un de ses analogues sont mis en œuvre dans le cadre de la présente invention dans une composition pour application labiale.

L'acide ascorbique et/ou au moins l'un de ses analogues peu(ven)t être présent(s) dans une composition pour application labiale conforme à la présente invention dans une teneur totale allant de 0,0001 à 20 % en poids, par exemple de 0,001 à 10 %, et de préférence de 0,01 à 10 % en poids par rapport au poids total de la composition.

Le présent texte décrit également, outre l'invention revendiquée, des compositions où l'acide ascorbique et/ou au moins l'un de ses analogues peu(ven)t être le(s) seul(s) actif(s) utile(s) à titre d'agent protecteur des lèvres fragiles.

Selon un mode de réalisation, les compositions selon l'invention peuvent contenir de l'acide ascorbique ou l'un de ses analogues à titre d'actif protecteur vis-à-vis des lèvres fragiles.

Comme indiqué précédemment, selon un autre mode de réalisation, la composition pour application labiale conforme à la présente invention contenant l'acide ascorbique et/ou au moins l'un de ses analogues peut en outre comprendre un ou plusieurs autres actifs cosmétiques et/ou dermatologiques qui sont détaillés ci-après dans la description qui suit. Ces actifs additionnels sont susceptibles de participer à la manifestation de l'effet recherché ou de procurer des effets complémentaires ou voire de tout autre effet pour autant qu'il ne gêne pas la manifestation de l'effet recherché, tel que défini ci-après

Le présent texte décrit également un procédé cosmétique de soin ou de traitement non thérapeutique des lèvres des êtres humains, comprenant l'application sur les lèvres d'une composition telle que définie ci-dessus, comprenant au moins de l'acide ascorbique ou au moins l'un de ses analogues.

Les utilisations et compositions selon l'invention mettent en œuvre conjointement au moins de l'acide ascorbique ou l'un de ses analogues et au moins la N-oléoyldihydrosphingosine et la α-hydroxypalmitoyldihydrosphingosine.

### ASSOCIATION D'UN PRECURSEUR DE CERAMIDES ET DE L'ACIDE ASCORBIQUE ET/OU SES ANALOGUES

Lorsqu'elle est appliquée au contact des lèvres, une composition comprenant de l'acide ascorbique ou au moins l'un de ses analogues va permettre de transformer de manière spécifique les précurseurs de céramides cibles présents naturellement dans les lèvres, ainsi que ceux apportés par la composition labiale selon l'invention, comprenant des précurseurs de céramide à base 6-hydroxy-4-sphingénine.

En apportant sur les lèvres l'acide ascorbique et ses analogues et le précurseur de céramide tel que défini précédemment, on met ainsi directement à la disposition des lèvres tous les composants nécessaires à l'obtention des céramides cibles. Ainsi, l'augmentation des céramides cibles ne se fera pas au détriment des précurseurs de céramide présents naturellement dans les lèvres bien qu'il ne soit pas exclu que ces derniers soient également mobilisés par les enzymes 6-hydroxylases. L'apport de précurseurs de céramide permet ainsi d'accéder plus rapidement et en plus grande quantité à une augmentation du contenu des lèvres en céramides cibles sans que la biosynthèse naturelle en amont des précurseurs de céramide cible soit un facteur limitant.

Ainsi, le présent texte décrit, outre l'invention revendiquée, l'utilisation cosmétique d'au moins l'acide ascorbique ou l'un de ses analogues associé à au moins un précurseur de céramide à base 6-hydroxy-4-sphingénine, ladite association étant dédiée à la protection des lèvres fragiles, notamment la zone des vermillons, et des signes cliniques associés, tels que définis précédemment.

Selon un mode de réalisation particulier, l'invention concerne une composition, pour application labiale, comprenant dans un milieu physiologiquement acceptable, au moins la N-oléoyldihydrosphingosine et la α-hydroxypalmitoyldihydrosphingosine associées à au moins de l'acide ascorbique ou au moins l'un de ses analogues tels que définis ci-dessus.

Une composition labiale selon cet aspect de l'invention peut notamment comprendre dans un milieu physiologiquement acceptable, de l'acide ascorbique ou au moins l'un de ses analogues associé à la N-oléoyldihydrosphingosine et la α-hydroxypalmitoyldihydrosphingosine, différentes des précurseurs de céramide naturellement présents dans les lèvres.

Selon un autre mode de réalisation, les utilisation et composition selon l'invention peuvent mettre en œuvre au moins de l'acide ascorbique ou l'un de ses analogues tels que définis ci-dessus et au moins la N-oléoyldihydrosphingosine et la α-hydroxypalmitoyldihydrosphingosine, en association avec un ou plusieurs autres actif additionnels tel que définis dans ce qui suit.

A titre d'actif cosmétique et/ou dermatologique, pouvant être mis en œuvre en association avec la N-oléoyldihydrosphingosine et la α-hydroxypalmitoyldihydrosphingosine et l'acide ascorbique ou au moins l'un de ses analogues tels qu'indiqués précédemment, on comprend une substance procurant une action au niveau du support d'application, en l'occurrence les lèvres, qui par un mécanisme d'action qui lui est propre, modifie sa structure et/ou son métabolisme. Certains de ces actifs sont notamment détaillés ci-dessous. Ainsi, il peut par exemple s'agir également d'une action protectrice des lèvres fragiles, mais aussi d'une action cicatrisante, apaisante, hydratante ou encore desquamante. Typiquement, le milieu physiologiquement acceptable tel que défini ci-après, ou encore les agents structurants, les polymères filmogènes et matières colorantes ne sont pas des actifs.

Ces actifs sont susceptibles de participer à la manifestation de l'effet recherché ou de procurer des effets complémentaires, voire de tout autre effet pour autant qu'ils ne gênent pas la manifestation de l'effet recherché.

Comme indiqué précédemment, une composition selon la présente invention, notamment une composition pour application labiale, peut en outre comprendre un ou plusieurs autres actifs cosmétiques et/ou dermatologiques qui sont détaillés dans ce qui suit.

### ACTIFS ADDITIONNELS

Les actifs additionnels susceptibles de procurer des effets complémentaires peuvent notamment appartenir à la famille des agents hydratants, des agents desquamants, des agents anti-pollution ou anti-radicalaire, des agents apaisants et des agents cicatrisants.

### Agents hydratants

Par « agent hydratant », on entend :
- soit un composé agissant sur la fonction barrière, en vue de maintenir l'hydratation du stratum corneum, ou un composé occlusif. On peut citer les céramides distincts des précurseurs de céramide considérés précédemment, les composés à base sphingoïde, les lécithines, les glycosphingolipides, les phospholipides, le cholestérol et ses dérivés, les phytostérols (stigmastérol, β-sitostérol, campestérol), les acides gras libres, les acides gras essentiels, le 1-2 diacylglycérol, la vaseline et la lanoline ;
- soit un composé augmentant directement la teneur en eau du stratum corneum, tel que le thréalose et ses dérivés, l'acide hyaluronique et ses dérivés, le glycérol, le pentanediol, le xylitol, le polyacrylate de glycérol, l'ectoïne et ses dérivés, le chitosane, les oligo- et polysaccharides comme le produit commercialisé sous la référence Pentavitin, les alginates (notamment le produit Sobalg PH 154 commercialisé par la société Grindsted).

En particulier, l'acide ascorbique ou l'un de ses analogues et la N-oléoyldihydrosphingosine et la α-hydroxypalmitoyldihydrosphingosine peuvent être associés à un facteur naturel d'hydratation dérivant de la dégradation de la filaggrine tel que défini ci-dessous.

Par « facteur naturel d'hydratation (ou NMF pour « natural moistering factor ») dérivant notamment de la dégradation de la filaggrine », on entend désigner au sens de l'invention un composé dérivant, c'est-à-dire issu directement ou indirectement, de la protéolyse de la filaggrine.

Parmi les NMFs dérivant de la dégradation de la filaggrine utiles selon l'invention, on peut notamment citer la sérine, le tryptophane, l'acide pyrrolidone carboxylique et l'acide urocanique, ainsi que leurs sels et leurs dérivés.

Par « dérivés », on entend désigner les isomères, les esters et les sels des esters de ces composés, lorsqu'ils existent.

Par exemple, la sérine considérée selon l'invention peut être choisie parmi la L-sérine, la D-sérine ou la (D,L)-sérine.

Selon un mode de réalisation, le (ou les) facteur(s) naturel(s) d'hydratation dérivant de la dégradation de la filaggrine peu(ven)t être choisi(s) parmi l'acide pyrrolidone carboxylique (PCA), ses sels, notamment de sodium, de lysine, d'arginine, d'aluminium, de magnésium, de cuivre, de zinc, de manganèse, et ses dérivés, par exemple, ses dérivés lipophiles, notamment le pyrrolidone carboxylate de menthyle, ses esters de C₂ à C₂₄, ramifiés ou linéaires, et plus particulièrement en C₈ à C₁₄, et notamment de lauryle, de cétyle, d'octyldodécyle, et de tétradécyle et les sels de ses esters comme par exemple de PCA de cocoyle et de sodium .

On peut notamment utiliser les esters de PCA d'octyldodécyle de tétradécyle commercialisés par la société SOLABIA respectivement sous les références Ceramidone^{®} et Myristidone^{®}, ou encore le PCA de cocoyle et de sodium commercialisé par la société ZSCHIMMER et SCHWARZ sous la référence protelan NMA^{®}.

Le présent texte décrit ainsi l'utilisation cosmétique de l'acide ascorbique ou d'au moins l'un de ses analogues à titre d'agent protecteur des lèvres fragiles, dans laquelle celui-ci est associé à un facteur naturel d'hydratation dérivant de la dégradation de la filaggrine.

En particulier, il décrit l'utilisation cosmétique dans laquelle l'acide ascorbique ou au moins l'un de ses analogues est associé à la sérine.

Il décrit également à l'utilisation cosmétique dans laquelle l'acide ascorbique ou au moins l'un de ses analogues est associé au tryptophane.

Ces agents actifs précités peuvent représenter de 0,001 % à 30 % et de préférence de 0,01 à 20% du poids total de la composition conforme à l'invention.

### Agents desquamants

Par « agent desquamant », on entend tout composé capable d'agir :
- soit directement sur la desquamation en favorisant l'exfoliation, tel que les β-hydroxyacides, en particulier l'acide salicylique et ses dérivés (dont l'acide n-octanoyl 5-salicylique) ; les α-hydroxyacides, tels que les acides glycolique, tartrique, malique ou mandélique ; l'acide gentisique ; les oligofucoses ; l'acide cinnamique ; l'extrait de Saphora japonica ; le resvératrol et certains dérivés d'acide jasmonique ;
- soit sur les enzymes impliquées dans la desquamation ou la dégradation des cornéodesmosomes, les glycosidases, la stratum corneum chymotryptic enzym (SCCE) voire d'autres protéases (trypsine, chymotrypsine-like). On peut citer les agents chélatant des sels minéraux : l'EDTA ; l'acide N-acyl-N,N',N' éthylène diaminetriacétique ; les composés aminosulfoniques et en particulier l'acide 4-(2-hydroxyéthyl)piperazine-1-propane sulfonique (HEPES) ; les dérivés de l'acide 2-oxothiazolidine-4-carboxylique (procystéine) ; les dérivés de sucre tels que l'O-octanoyl-6-D-maltose et la N-acétyl glucosamine.

### Agent anti-pollution ou anti-radicalaire

Par l'expression « agent anti-pollution », on entend tout composé capable de piéger l'ozone, les composés aromatiques mono- ou polycycliques tels que le benzopyrène et/ou les métaux lourds tels que le cobalt, le mercure, le cadmium et/ou le nickel. Par « agent anti-radicalaire », on entend tout composé capable de piéger les radicaux libres.

Comme agents piégeurs d'ozone utilisables dans la composition selon l'invention, on peut citer en particulier ; les phénols et polyphénols, en particulier les tannins, l'acide ellagique et l'acide tannique ; l'épigallocatéchine et les extraits naturels en contenant ; les extraits de feuille d'olivier ; les extraits de thé, en particulier de thé vert ; les anthocyanes ; les extraits de romarin ; les acides phénols, en particulier l'acide chorogénique ; les stilbènes, en particulier le resvératrol ; des chélatants comme la N,N'-bis-(3,4,5-triméthoxybenzyl)éthylènediamine ou l'un de ses sels, complexes métalliques ou esters ; des caroténoïdes tels que la crocétine.

Comme agents piégeurs de composés aromatiques mono- ou polycycliques utilisables dans la composition selon l'invention, on peut citer en particulier les tannins tels que l'acide ellagique ; les dérivés indoles, en particulier l'indol-3-carbinol ; les extraits de thé en particulier de thé vert, les extraits de Jacinthe d'eau ou eichornia crassipes.

Enfin, comme agents piégeurs de métaux lourds utilisables dans la composition selon l'invention, on peut citer en particulier les agents chélatants tels que l'EDTA, le sel pentasodique d'éthylènediamine tétraméthylène phosphonique, et la N,N'-bis-(3,4,5-triméthoxybenzyl)éthylènediamine ou l'un de ses sels, complexes métalliques ou esters ; l'acide phytique ; les dérivés de chitosan ; les extraits de thé, en particulier de thé vert ; les tannins tels que l'acide ellagique.

Les agents anti-radicalaires utilisables dans la composition selon l'invention comprennent, outre certains agents anti-pollution mentionnés précédemment, la vitamine E et ses dérivés tels que l'acétate de tocophéryle ; les bioflavonoïdes ; le co-enzyme Q10 ou ubiquinone ; certaines enzymes comme la catalase, le superoxyde dismutase et les extraits de germes de blé en contenant, la lactoperoxydase, le glutathion peroxydase et les quinones réductases ; le glutathion ; le benzylidène camphre ; les benzylcyclanones ; les naphtalénones substituées ; le phytantriol ; le gamma-oryzanol ; la guanosine ; les lignanes et la mélatonine.

### Agents apaisants

Comme agents apaisants utilisables dans la composition conforme à l'invention, on peut citer : les triterpènes pentacycliques et les extraits de plantes (ex : Glycyrrhiza glabra) en contenant comme l'acide β-glycyrrhétinique et ses sels et/ou ses dérivés (l'acide glycyrrhétinique monoglucuronide, le stearyl glycyrrhetinate, l'acide 3- stéaroyloxy glycyrrhetique), l'acide ursolique et ses sels, l'acide oléanolique et ses sels, l'acide bétulinique et ses sels, les extraits de plantes telles que *Paeonia suffruticosa* et / ou *lactiflora, Laminaria saccharina, Boswellia serrata, Centipeda cunnighami, Helianthus annuus, Linum usitatissimum, Cola nitida, Epilobium Angustifolium, Aloe vera, Bacopa monieri,* les sels de l'acide salicylique et en particulier le salicylate de zinc, l'huile de Canola, le bisabolol et les extraits de camomille, l'allantoïne, le Sépivital EPC (diesterphosphorique de vitamine E) de Seppic, les huiles insaturées en oméga 3 telles que les huiles de rosier muscat, de cassis, d'ecchium, ou de poisson, des extraits de plancton, les tocotrienols, le piperonal, un extrait de clou de girofle, les phytostérols, la cortisone, l'hydrocortisone, l'indométhacine et la beta méthasone.

### Agents cicatrisants

Des exemples d'agents cicatrisants sont notamment l'extrait de feuilles de fougère commercialisé sous la référence Mamaku Vital Essence^{®} par Lucas Meyer, les peptides de riz obtenus par hydrolyse de protéines de riz commercialisés sous la dénomination Nutripeptide^{®} par Silab, l'oxyde de zinc, la vitamine B5.

Parmi les actifs ne procurant pas d'effet complémentaire mais procurant d'autres effets que l'effet protecteur des lèvres fragiles, pour autant qu'ils ne gênent pas la manifestation de l'effet recherché, on peut notamment citer :
- les composés favorisant la microcirculation sanguine tels que les agents favorisant la production de monoxyde d'azote ; les agents anti-hypertenseurs en particulier les ouvreurs de canaux potassium ; les agents inhibiteurs de phosphodiestérases ; les flavonoïdes ou les flavoglycosides ; les glucosides tels que le C-β-D-xylopyranoside-2-hydroxy-propane ; les extraits végétaux aux propriétés vasodilatatrices ; d'autres agents vasodilatateurs et les agents modulateurs de la température,
- les composés myorelaxants tels que le gluconate de magnésium, le gluconate de manganèse et l'extrait de Rose.

A titre d'autres associations particulières pouvant être mises en œuvre dans le cadre de la présente invention, on peut également citer l' association d'un actif permettant d'augmenter la teneur en céramides telle que définie selon la présente invention, notamment choisi parmi l'acide ascorbique ou au moins l'un de ses analogues ou la N-oléoyldihydrosphingosine ou la α-hydroxypalmitoyldihydrosphingosine, avec au moins un actif additionnel choisi parmi les extraits de Centella Asiatica, le madécassoside, la vitamine B5, l'hydroxyéthyl urée, l'oxyde de zinc et le C-β-D-*xylopyranoside-2-hydroxy-propane.*

Le ou les actifs additionnels pouvant être associé(s) à la N-oléoyldihydrosphingosine et à la α-hydroxypalmitoyldihydrosphingosineet à l'acide ascorbique ou ses analogues, peuvent être présents dans une teneur totale allant de 0,001 à 30 % en poids, notamment de 0,01 à 20 % en poids par rapport au poids total de la composition.

D'une manière générale, l'acide ascorbique et/ou l'un de ses analogues ou le précurseur de 6-hydroxy-4-sphingenine, en association ou non avec un autre actif, notamment tel que défini précédemment, est mis en œuvre dans un milieu physiologiquement acceptable sous la forme d'une composition.

### MILIEU PHYSIOLOGIQUEMENT ACCEPTABLE

Par « milieu physiologiquement acceptable », on entend un milieu compatible avec les lèvres, comme les huiles ou les solvants organiques couramment employés dans les compositions cosmétiques.

Ces huiles peuvent être présentes en une teneur allant de 0,01 à 90 %, et mieux de 0,1 à 85 % en poids, par rapport au poids total de la composition.

Le milieu physiologiquement acceptable de la composition selon l'invention peut également comprendre un ou plusieurs solvants organiques, physiologiquement acceptables (tolérance, toxicologie et toucher acceptables).

Le solvant organique non aqueux est de préférence un composé non soluble dans l'eau et liquide à température ambiante et pression atmosphérique.

Le milieu physiologiquement acceptable peut comprendre, un milieu hydrophile comprenant de l'eau ou un mélange d'eau et de solvant(s) organique(s) hydrophile(s) comme les alcools et notamment les monoalcools inférieurs linéaires ou ramifiés ayant de 2 à 5 atomes de carbone comme l'éthanol, l'isopropanol ou le n-propanol, la diglycérine, le propylène glycol, le sorbitol, le penthylène glycol, et les polyéthylène glycols, ou bien encore des éthers en C₂ et des aldéhydes en C₂-C₄ hydrophiles.

La composition selon l'invention peut également être sensiblement exempte d'un tel milieu hydrophile. La composition selon l'invention peut comprendre moins de 5 % en poids, voire moins de 2 % en poids d'eau par rapport au poids total de la composition et peut en particulier être anhydre.

### PHASE SOLVANT

La phase solvant non aqueuse, formée par les huiles et solvants est susceptible de former une phase continue.

Par "composé volatil", on entend, au sens de l'invention, tout composé (ou milieu non aqueux) susceptible de s'évaporer au contact des lèvres en moins d'une heure, à température ambiante et pression atmosphérique. Le composé volatil est un composé cosmétique volatil, liquide à température ambiante, ayant notamment une pression de vapeur non nulle, à température ambiante et pression atmosphérique, notamment ayant une pression de vapeur allant de 0,13 Pa à 40 000 Pa (10⁻³ à 300 mm de Hg), en particulier allant de 1,3 Pa à 13 000 Pa (0,01 à 100 mm de Hg), et plus particulièrement allant de 1,3 Pa à 1300 Pa (0,01 à 10 mm de Hg).

Par opposition, on entend par "composé non volatil", un composé restant sur les lèvres à température ambiante et pression atmosphérique, au moins plusieurs heures et ayant notamment une pression de vapeur inférieure à 10⁻³ mm de Hg (0,13Pa).

Le composé volatil, non soluble dans l'eau et liquide à température ambiante est en particulier une huile (corps gras liquide à 25°C et pression atmosphérique) ou un solvant organique physiologiquement acceptable.

Les huiles volatiles peuvent être des huiles hydrocarbonées, des huiles siliconées, des huiles fluorées ou leurs mélanges.

On entend par "huile hydrocarbonée", une huile contenant principalement des atomes d'hydrogène et de carbone et éventuellement des atomes d'oxygène, d'azote, de soufre, de phosphore. Les huiles hydrocarbonées volatiles peuvent être choisies parmi les huiles hydrocarbonées ayant de 8 à 16 atomes de carbones, et notamment les alcanes ramifiés en C₈-C₁₆ comme les isoalcanes en C₈-C₁₆ d'origine pétrolière (appelées aussi isoparaffines) comme l'isododécane (encore appelé 2,2,4,4,6-pentaméthylheptane), l'isodécane, l'isohexadécane, et par exemple les huiles vendues sous les noms commerciaux "d'Isopars^{®}" ou de "Permetyls^{®} ", les esters ramifiés en C₈-C₁₆, le néopentanoate d'iso-hexyle, et leurs mélanges. D'autres huiles hydrocarbonées volatiles comme les distillats de pétrole, notamment ceux vendus sous la dénomination "Shell Solt^{®}" par la société SHELL, peuvent aussi être utilisées.

Comme huiles volatiles, on peut aussi utiliser les silicones volatiles, comme par exemple les huiles de silicones linéaires ou cycliques volatiles, notamment celles ayant une viscosité inférieure à 8 centistokes, et ayant notamment de 2 à 10 atomes de silicium, ces silicones comportant éventuellement des groupes alkyles ou alkoxy ayant de 1 à 22 atomes de carbone. Comme huile de silicone volatile utilisable dans l'invention, on peut citer notamment l'octaméthyl cyclotétrasiloxane, le décaméthyl cyclopentasiloxane, le dodécaméthyl cyclohexasiloxane, l'heptaméthyl hexyltrisiloxane, l'heptaméthyloctyl trisiloxane, l'hexaméthyl disiloxane, l'octaméthyl trisiloxane, le décaméthyl tétrasiloxane, le dodécaméthyl pentasiloxane et leurs mélanges.

La phase solvant non aqueuse peut également comprendre au moins un composé non volatil, non soluble dans l'eau et liquide à température ambiante, notamment au moins une huile non volatile, qui peut être en particulier choisie parmi les huiles hydrocarbonées et/ou siliconées et/ou fluorées non volatiles et de préférence brillantes.

Comme huile hydrocarbonée non volatile, on peut notamment citer :
- les huiles hydrocarbonées d'origine végétale telles que les triglycérides constitués d'esters d'acides gras et de glycérol dont les acides gras peuvent avoir des longueurs de chaînes variées de C₄ à C₂₄, ces derniers pouvant être linéaires ou ramifiées, saturées ou insaturées ; ces huiles sont notamment les huiles de germe de blé, de tournesol, de pépins de raisin, de sésame, de maïs, d'abricot, de ricin, de karité, d'avocat, d'olive, de soja, l'huile d'amande douce, de palme, de colza, de coton, de noisette, de macadamia, de jojoba, de luzerne, de pavot, de potimarron, de sésame, de courge, de colza, de cassis, d'onagre, de millet, d'orge, de quinoa, de seigle, de carthame, de bancoulier, de passiflore, de rosier muscat ; ou encore les triglycérides des acides caprylique/caprique comme ceux vendus par la société Stéarineries Dubois ou ceux vendus sous les dénominations de "Miglyol 810^{®}" , "812^{®} " et "818^{®} " par la société Dynamit Nobel ;
- les éthers de synthèse ayant de 10 à 40 atomes de carbone ;
- les hydrocarbures linéaires ou ramifiés, d'origine minérale ou synthétique tels que la vaseline, les polydécènes, le polyisobutène hydrogéné tel que le parléam, le squalane, et leurs mélanges ;
- les esters de synthèse comme les huiles de formule R₁COOR₂ dans laquelle R₁ représente le reste d'un acide gras linéaire ou ramifié comportant de 1 à 40 atomes de carbone et R₂ représente une chaîne hydrocarbonée notamment ramifiée contenant de 1 à 40 atomes de carbone à condition que R₁ + R₂ soit ≥ 10, comme par exemple l'huile de Purcellin (octanoate de cétostéaryle), le myristate d'isopropyle, le palmitate d'isopropyle, le benzoate d'alcool en C₁₂ à C₁₅, le laurate d'hexyle, l'adipate de diisopropyle, l'isononanoate d'isononyle, le palmitate de 2-éthyl-hexyle, l'isostéarate d'isostéarate, des octanoates, décanoates ou ricinoléates d'alcools ou de polyalcools comme le dioctanoate de propylène glycol ; les esters hydroxylés comme le lactate d'isostéaryle, le malate de di-isostéaryle ; et les esters du pentaérythritol ;
- les alcools gras liquides à température ambiante à chaîne carbonée ramifiée et/ou insaturée ayant de 12 à 26 atomes de carbone comme l'octyl dodécanol, l'alcool isostéarylique, l'alcool oléique, le 2-hexyldécanol, le 2-butyloctanol, le 2-undécylpentadécanol ;
- les acides gras supérieurs tels que l'acide oléique, l'acide linoléique, l'acide linolénique et leurs mélanges.

Les huiles de silicone non volatiles utilisables selon l'invention peuvent être les polydiméthylsiloxanes (PDMS) non volatiles, les polydiméthylsiloxanes comportant des groupements alkyle ou alcoxy, pendant et/ou en bout de chaîne siliconée, groupements ayant chacun de 2 à 24 atomes de carbone, les silicones phénylées comme les phényl triméthicones, les phényl diméthicones, les phényl triméthylsiloxy diphénylsiloxanes, les diphényl diméthicones, les diphényl méthyldiphényl trisiloxanes, les 2-phényléthyl triméthylsiloxysilicates.

Les huiles fluorées utilisables dans la composition conforme à l'invention sont notamment des huiles fluorosiliconées, des polyéthers fluorés, des silicones fluorées telles que décrites dans le document EP-A-847752.

### AGENT EPAISSISSANT/ STRUCTURANT

Les compositions conformes à l'invention peuvent se présenter sous forme épaissie notamment anhydre, par exemple sous la forme d'un stick. Elles peuvent être épaissies avec au moins un agent épaississant choisi parmi les gélifiants de phase grasse, les cires, les corps gras pâteux, les charges et leurs mélanges.

Comme gélifiant de phase grasse, on peut citer les argiles éventuellement modifiées comme les hectorites modifiées par un chlorure d'ammonium d'acide gras en C₁₀ à C₂₂, comme l'hectorite modifiée par du chlorure de di-stéaryl di-méthyl ammonium ; la silice ; les organopolysiloxanes élastomériques partiellement ou totalement réticulés, de structure tridimensionnelle, comme ceux commercialisés sous les noms KSG6, KSG16, KSG18 de Shin-Etsu, Trefil E-505C ou Trefil E-506C de Dow-Corning, Gransil SR-CYC, SR DMF10, SR-DC556, SR 5CYC gel, SR DMF 10 gel, SR DC 556 gel de Grant Industries, SF 1204 et JK 113 de General Electric ; les galactommananes comportant un à six et mieux de deux à quatre groupes hydroxyle par ose, substitués par une chaîne alkyle saturée ou non, comme la gomme de guar alkylée par des chaînes alkyle en C₁ à C₆ et mieux en C₁ à C₃ et plus particulièrement la guar éthylée ayant un degré de substitution de 2 à 3 telle que celle vendue par la société Aqualon sous le nom N-HANCE-AG ; les gommes notamment siliconées comme les polydiméthylsiloxane PDMS ayant une viscosité > 500 000 centistokes et/ou un poids moléculaire supérieur ou égal à 200 000 g/mol ; les polyamides siliconés.

On peut également utiliser les polyamides siliconés du type polyorganosiloxane tels que ceux décrits dans les documents US-A-5,874,069, US-A-5,919,441, US-A-6,051,216 et US-A-5,981,680.

Ces polymères siliconés peuvent appartenir aux deux familles suivantes :
- des polyorganosiloxanes comportant au moins deux groupes capables d'établir des interactions hydrogène, ces deux groupes étant situés dans la chaîne du polymère, et/ou
- des polyorganosiloxanes comportant au moins deux groupes capables d'établir des interactions hydrogène, ces deux groupes étant situés sur des greffons ou ramifications.

Ces gélifiants sont utilisés par exemple à des concentrations de 0,05 à 15 % du poids total de la composition.

Les compositions conformes à l'invention peuvent contenir au moins une cire.

Par "cire" au sens de la présente invention, on entend un composé gras lipophile, solide à température ambiante (25 °C), à changement d'état solide/liquide réversible, ayant une température de fusion supérieure à 30 °C pouvant aller jusqu'à 200 ° C, une dureté supérieure à 0,5 MPa, et présentant à l'état solide une organisation cristalline anisotrope. En portant la cire à sa température de fusion, il est possible de la rendre miscible aux huiles et de former un mélange homogène microscopiquement, mais en ramenant la température du mélange à la température ambiante, on obtient une recristallisation de la cire dans les huiles du mélange.

Les cires utilisables dans l'invention sont des composés solides à température ambiante, destinés à structurer la composition en particulier sous forme de stick ; elles peuvent être hydrocarbonées, fluorées et/ou siliconées et être d'origine végétale, minérale, animale et/ou synthétique. En particulier, elles présentent une température de fusion supérieure à 40 °C et mieux supérieure à 45 °C.

Comme cire utilisable dans l'invention, on peut citer celles généralement utilisées dans le domaine cosmétique : elles sont notamment d'origine naturelle comme la cire d'abeilles, la cire de Carnauba, de Candelilla, d'Ouricoury, du Japon, de fibres de liège ou de canne à sucre, de riz, de Montan, la paraffine, les cires de lignite ou microcristalline, la cérésine ou l'ozokérite, les huiles hydrogénées comme l'huile de jojoba ; les cires synthétiques comme les cires de polyéthylène issues de la polymérisation ou copolymérisation de l'éthylène et les cires de Fischer-Tropsch ou encore des esters d'acides gras comme l'octacosanyl stéarate, les glycérides concrets à 40 °C et mieux à 45 °C, les cires de silicones comme les alkyl- ou alkoxydiméthicones ayant une chaîne alkyle ou alcoxy de 10 à 45 atomes de carbone, les esters de poly(di)méthylsiloxane solide à 40 °C dont la chaîne ester comporte au moins 10 atomes de carbone ; et leurs mélanges.

Les compositions conformes à l'invention contiennent avantageusement de la cire de polyéthylène de masse moléculaire en poids comprise entre 300 et 700 g/mol, notamment égale à 500 g/mol.

A titre indicatif, la cire peut représenter de 0,01 à 50 %, de préférence de 2 à 40 %, et mieux de 5 à 30 % du poids total de la composition.

Les compositions conformes à l'invention peuvent également contenir au moins un composé pâteux.

Par "pâteux" au sens de la présente invention, on entend désigner un composé gras lipophile, à changement d'état solide/liquide réversible, et comportant à la température de 23 °C une fraction liquide et une fraction solide. On entend également par "pâteux", le polylaurate de vinyle.

Le composé pâteux est avantageusement choisi parmi :
- les composés fluorés polymères ou non,
- les composés siliconés polymères ou non,
- les polymères vinyliques,
- les homopolymères d'oléfines,
- les copolymères d'oléfines,
- les homopolymères et copolymères de diènes hydrogénés,
- les oligomères linéaires ou ramifiés, homo ou copolymères de (méth)acrylates d'alkyles ayant de préférence un groupement alkyle en C₈-C₃₀,
- les oligomères homo et copolymères d'esters vinyliques ayant des groupements alkyles en C₈-C₃₀,
- les oligomères homo et copolymères de vinyléthers ayant des groupements alkyles en C₈-C₃₀,
- les polyéthers liposolubles résultant de la polyéthérification entre un ou plusieurs diols en C₂-C₁₀₀, de préférence en C₂-C₅₀,
- les esters, et
- leurs mélanges.

Parmi les polyéthers liposolubles, on préfère en particulier les copolymères d'éthylène-oxyde et/ou de propylène-oxyde avec des alkylènes-oxydes à longue chaîne en C₆-C₃₀, de préférence encore tels que le rapport pondéral de l'éthylène-oxyde et/ou de propylène-oxyde avec alkylènes-oxydes dans le copolymère est de 5:95 à 70:30. Dans cette famille, on citera notamment les copolymères tels que les alkylènes-oxydes à longue chaîne sont disposés en blocs ayant un poids moléculaire moyen de 1000 à 10000, par exemple un copolymère bloc de polyoxyethylène/polydodécyle glycol tel que les éthers de dodécanediol (22 mol) et de polyéthylène glycol (45 OE) commercialisés sous la marque ELFACOS ST9 par Akzo Nobel.

Parmi les pâteux esters, on préfère notamment :
- les esters d'un glycérol oligomère, notamment les esters de diglycérol, en particulier les condensats d'acide adipique et de glycérol, pour lesquels une partie des groupes hydroxyle des glycérols ont réagi avec un mélange d'acides gras tels que l'acide stéarique, l'acide caprique, l'acide stéarique et l'acide isostéarique et l'acide 12-hydroxystéarique, à l'image notamment de ceux commercialisé sous la marque Softisan 649 par la société Sasol
- le propionate d'arachidyle commercialisé sous la marque Waxenol 801 par Alzo,
- les esters de phytostérol,
- les polyesters non réticulés résultant de la polycondensation entre un diacide ou un polyacide carboxylique linéaire ou ramifié en C₄-C₅₀ et un diol ou un polyol en C₂-C₅₀, différent du polyester décrit précédemment,
- les esters aliphatiques d'ester résultant de l'estérification d'un ester d'acide hydroxycarboxylique aliphatique par un acide monocarboxylique aliphatique, et leurs mélanges, comme
   - l'ester résultant de la réaction d'estérification de l'huile de ricin hydrogénée avec l'acide isostéarique dans les proportions 1 pour 1 (1/1) ou monoisostéarate d'huile de ricin hydrogénée,
   - l'ester résultant de la réaction d'estérification de l'huile de ricin hydrogénée avec l'acide isostéarique dans les proportions 1 pour 2 (1/2) ou le diisostéarate d'huile de ricin hydrogénée,
   - l'ester résultant de la réaction d'estérification de l'huile de ricin hydrogénée avec l'acide isostéarique dans les proportions 1 pour 3 (1/3) ou triisostéarate d'huile de ricin hydrogénée,
   - et leurs mélanges.

Parmi les composés pâteux d'origine végétale, on choisira de préférence un mélange de stérols de soja et de pentaérythritol oxyéthyléné (5OE) oxypropyléné (5OP), commercialisé sous la référence Lanolide par la société VEVY.

Le composé pâteux représente de préférence 1 à 99 %, mieux 1 à 60 %, mieux 2 à 30 % et mieux encore 5 à 20 % en poids de la composition.

Comme précisée précédemment, les compositions selon l'invention peuvent comprendre également une ou plusieurs charges, notamment en une teneur allant de 0,01 % à 50 % en poids, par rapport au poids total de la composition, de préférence allant de 0,01 % à 30 % en poids.

Par charges, il faut comprendre des particules de toute forme, incolores ou blanches, minérales ou de synthèse, insolubles dans le milieu de la composition quelle que soit la température à laquelle la composition est fabriquée. Ces charges servent notamment à modifier la rhéologie ou la texture de la composition.

Les charges peuvent être minérales ou organiques de toute forme, plaquettaires, sphériques ou oblongues. On peut citer le talc, le mica, la silice, le kaolin, les poudres de polyamide (Nylon^{®})(Orgasol^{®} de chez Atochem), de poly-β-alanine et de polyéthylène, les poudres de polymères de tétrafluoroéthylène (Téflon^{®}), la lauroyl-lysine, l'amidon, le nitrure de bore, les microsphères creuses polymériques telles que celles de chlorure de polyvinylidène/acrylonitrile comme l'Expancel^{®} (Nobel Industrie), de copolymères d'acide acrylique (Polytrap^{®} 603 de la société Dow Corning) et les microbilles de résine de silicone (Tospearls^{®} de Toshiba, par exemple), les particules de polyorganosiloxanes élastomères, le carbonate de calcium précipité, le carbonate et l'hydro-carbonate de magnésium, l'hydroxyapatite, les microsphères de silice creuses (Silica Beads^{®} de Maprecos), les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium, le Polypore^{®} L 200 (Chemdal Corporation). On peut encore citer les charges à base de silice comme l'Aerosil 200, l'Aerosil 300 ; le Sunsphare L-31, le Sunphare H-31 commercialisés par Asahi Glass ; le Chemicelen commercialisé par Asahi Chemical ; les composites de silice et de dioxyde de titane comme la série TSG commercialisée par Nippon Sheet Glass. Enfin, on peut citer les poudres de poyuréthanne, en particulier les poudres de polyuréthanne réticulé comprenant un copolymère, ledit copolymère comprenant du triméthylol hexyllactone. En particulier, il peut s'agir d'un polymère d'hexaméthylène di-isocyanate/triméthylol hexyllactone. De telles particules sont notamment disponibles dans le commerce, par exemple sous la dénomination de PLASTIC POWDER D-400^{®} ou PLASTIC POWDER D-800^{®} de la société TOSHIKI.

### MATIERE COLORANTE

Selon un mode de réalisation particulier, les compositions conformes à l'invention comprennent au moins une matière colorante qui peut être choisie parmi les colorants, les pigments, les nacres et leurs mélanges. Cette matière colorante peut représenter de 0,001 à 98 %, de préférence de 0,1 à 85 % et notamment de 0,5 à 60 % du poids total de la composition.

Pour des raisons évidentes, ces matières colorantes sont mises en œuvre dans les compositions conformes à l'invention, de manière à ne pas porter préjudice à l'effet plus particulièrement recherché selon l'invention et qui vise notamment à procurer une protection des lèvres fragiles.

Pour une composition sous forme de pâte ou coulée telle que les rouges à lèvres, on utilise en général de 0,5 à 50 % de matière colorante, de préférence de 2 à 40 % et notamment de 5 à 30 %, par rapport au poids total de la composition.

Les colorants sont de préférence des colorants liposolubles, bien que les colorants hydrosolubles puissent être utilisés. Les colorants liposolubles sont par exemple le rouge Soudan, le D & C Red 17, le D & C Green 6, le β-carotène, l'huile de soja, le brun Soudan, le D & C Yellow 11, le D & C Violet 2, le D & C orange 5, le jaune quinoléine, le rocou. Ils peuvent représenter de 0 à 20 % du poids de la composition et mieux de 0,1 à 6 %. Les colorants hydrosolubles sont notamment le jus de betterave, le bleu de méthylène et peuvent représenter de 0,01 à 6 % en poids de la composition (si présents).

Par pigments, il faut comprendre des particules blanches ou colorées, minérales ou organiques, insolubles dans la phase grasse liquide, destinées à colorer et/ou opacifier la composition. Par nacres, il faut comprendre des particules irisées, notamment produites par certains mollusques dans leur coquille ou bien synthétisées.

Les pigments peuvent être présents dans la composition à raison de 0,05 à 30 % du poids de la composition finale, et de préférence à raison de 0,1 à 20 %. Comme pigments minéraux utilisables dans l'invention, on peut citer les oxydes de titane, de zirconium ou de cérium ainsi que les oxydes de zinc, de fer ou de chrome et le bleu ferrique. Parmi les pigments organiques utilisables dans l'invention, on peut citer le noir de carbone, et les laques de baryum, strontium, calcium (D & C Red N°7), aluminium.

Les nacres peuvent être présentes dans la composition à raison de 0,001 à 20 % du poids total de la composition, de préférence à un taux de l'ordre de 1 à 15 %. Parmi les nacres utilisables dans l'invention, on peut citer le mica recouvert d'oxyde de titane, d'oxyde de fer, de pigment naturel ou d'oxychlorure de bismuth tel que le mica titane coloré.

Les compositions peuvent également contenir avantageusement des pigments gonio-chromatiques, par exemple des pigments multicouches interférentiels, et/ou des pigments réfléchissants. Ces deux types de pigments sont décrits dans le document FR 0 209 246 dont le contenu est incorporé par référence dans la présente demande.

### POLYMERE FILMOGENE

La composition peut comprendre en outre un polymère filmogène.

Selon la présente invention, on entend par « polymère filmogène », un polymère apte à former à lui seul ou en présence d'un agent auxiliaire de filmification, un film continu et adhérent sur les lèvres.

Parmi les polymères filmogènes utilisables dans la composition conforme à la présente invention, on peut citer les polymères synthétiques, de type radicalaire ou de type polycondensat, les polymères d'origine naturelle et leurs mélanges. Comme polymère filmogène, on peut citer en particulier les polymères acryliques, les polyuréthanes, les polyesters, les polyamides, les polyurées, les polymères cellulosiques comme la nitrocellulose.

Le polymère peut être associé à un ou des agents auxiliaires de filmification. Un tel agent de filmification peut être choisi parmi tous les composés connus de l'homme du métier comme étant susceptibles de remplir la fonction recherchée, et notamment être choisi parmi les agents plastifiants et les agents de coalescence.

La composition conforme à l'invention peut comprendre, en outre, tout ingrédient usuellement utilisé dans le domaine du soin des lèvres, tel que des huiles essentielles, des conservateurs, des parfums, des neutralisants, des polymères liposolubles notamment hydrocarbonés tels que les polyalkylènes ou le polylaurate de vinyle, des gélifants de phase aqueuse, des tensioactifs, des émollients, des tenseurs, des vitamines, des acides gras essentiels, des filtres solaires lipophiles ou hydrophiles, et leurs mélanges.

Bien entendu l'homme du métier veillera à choisir les éventuels ingrédients complémentaires et/ou leur quantité de telle manière que les propriétés avantageuses de la composition conforme à l'invention ne soient pas, ou substantiellement pas, altérées par l'adjonction envisagée.

Une composition de l'invention peut se présenter sous forme de pâte, notamment de pâte souple, de solide, de crème, de mousse, de spray, de poudre ou même de liquide. Elle peut être une émulsion huile-dans-eau (H/E), huile-dans-polyol, eau-dans-huile (E/H), eau-dans-polyol, une émulsion multiple (E/H/E ou polyol/H/E ou H/E/H), un gel anhydre, un solide ou une pâte souple, une phase huileuse liquide, une suspension, une dispersion ou une solution. De préférence, elle se présente sous forme de composition solide anhydre, et plus spécialement sous forme de stick.

L'homme du métier pourra choisir la forme galénique appropriée, ainsi que sa méthode de préparation, sur la base de ses connaissances générales, en tenant compte d'une part de la nature des constituants utilisés, notamment de leur solubilité dans le support, et d'autre part de l'application envisagée pour la composition.

Selon un mode de réalisation particulier, une composition conforme à l'invention peut se présenter sous forme épaisse anhydre. Aussi, l'invention se rapporte plus spécialement à une composition anhydre de maquillage ou de soin des lèvres fragiles, épaissie contenant au moins un agent épaississant choisi parmi les gélifiants de phase grasse, les cires, les charges et leurs mélanges tels que détaillés ci-après.

Une composition conforme à l'invention peut se présenter sous la forme d'une composition dermatologique ou de soin des lèvres, ou sous forme d'une composition de protection solaire des lèvres. Elle se présente alors sous forme non colorée, contenant éventuellement des actifs cosmétiques ou dermatologiques, autres que le précurseur de céramide, tels que détaillés ci-après. Elle peut alors être utilisée sous la forme de base de soin pour les lèvres fragiles. Elle peut notamment prendre la forme d'un baume à lèvres, notamment protégeant les lèvres des facteurs intrinsèques et extérieurs susceptibles de favoriser la survenue des lèvres fragiles. Parmi ces facteurs extérieurs on peut notamment citer le froid, les rayonnements UV, le vent et les stimulations physico-chimiques telles que la prise des repas, les rouges à lèvres, le tabagisme et la langue.

La composition conforme à l'invention peut également se présenter sous la forme d'un produit coloré de maquillage des lèvres, comme un rouge à lèvres ou un brillant à lèvres ou gloss, présentant des propriétés de soin et/ou de traitement.

Bien entendu la composition conforme à l'invention doit être cosmétiquement ou dermatologiquement acceptable, à savoir non toxique et susceptible d'être appliquée sur les lèvres d'êtres humains.

Selon un autre mode de réalisation, une composition conforme à l'invention peut se présenter sous une forme compatible avec une administration buccale. Selon cette alternative, elle est appliquée sur les lèvres par mise en contact de celle-ci avec la salive la contenant.

La présente invention a encore pour objet une composition selon pour son utilisation dans le traitement des signes localisés sur les vermillons des lèvres associés aux lèvres fragiles choisis parmi :
- la sensation d'inconfort et/ou de picotements ;
- la sensation de tiraillements ;
- la présence de squames ;
- les plaques sèches ;
- les fendillements ;
- les fissures ; et/ou
- les saignements,
la N-oléoyldihydrosphingosine et la α-hydroxypalmitoyldihydrosphingosine étant de conformation érythro (2S, 3R).

Les exemples suivants sont hors invention.

Les pourcentages sont donnés en poids.

### Exemple 1 :

Stick de rouge à lèvres coloré pour lèvres fragiles

| | |
|---|---|
| Huile de ricin | 3% |
| Huile de vaseline | 9% |
| Lanoline | 15% |
| Cire d'abeilles | 9% |
| Ozokérite | 10% |
| α-hydroxypalmitoyldihydrosphingosine | 0,24% |
| Palmitate d'ascorbyle | 0,2% |
| Lécithine de soja | 7% |
| Glycérine | 8% |
| Talc | 3% |
| D&C RED 27 | 5% |
| D&C RED 7 | 6% |
| Oxyde de fer noir | 2% |
| Polybutène | qsp 100% |
| parfum | qs |

### Exemple 2 :

Stick à lèvres non coloré pour lèvres fragiles

| | |
|---|---|
| Huile de ricin | 20% |
| Huile de jojoba | qsp 100% |
| Lanolate d'isopropyle | 28% |
| Cire microcristalline | 8% |
| Cire de carnauba | 8% |
| Phosphate de tri-oléyle | 1% |
| 2-oléoylamino-1,3-octadécanediol | 0,5% |

### Exemple 3 :

Crème hydratante pour lèvres fragiles

| | |
|---|---|
| Eau | qsp 100% |
| α-hydroxypalmitoyldihydrosphingosine | 0,2% |
| méthylparabène | 0,2% |
| Tristéarate de sorbitan | 1,4% |
| Alcool cétylique | 5,5% |
| Stéarate de glycéryle | 4,8% |
| Stéarate de polyéthylène glycol (40OE) | 3% |
| Huile de parléam | 23% |
| Cétyl phosphate de potassium | 1% |
| Parfum + colorant | qs |

### Exemple 4 :

Baume confort

| | |
|---|---|
| Triglycéride en C₂₄ | 10% |
| Dimèredilinoléate de dioctyldodécyle | 24% |
| Octyldodécanol | 15% |
| Triglycéride d'acide caprilique/caprylique | 10% |
| Huile de lanoline | 12% |
| Lanoline propoxylée | 6% |
| Lanoline acétylée | 6% |
| Cire de polyéthylène | 10% |
| Octaconasylstéarate | 5% |
| Oxyde de zinc | 0,5% |
| α-hydroxypalmitoyldihydrosphingosine | 0,2% |
| Palmitate d'ascorbyle | 0,2% |
| Colorant et parfum | qs |

### Exemple 5 :

Stick réparateur lèvres sensibles

| | |
|---|---|
| - hectorite modifiée par du chlorure de di-stearyl di-methyl ammonium | 0,5 % |
| - lanoline liquide | 27,2 % |
| - cire microcristalline | 10,5 % |
| - cire d'abeille polyglycérolée (3 moles) | 4,2 % |
| - lanoline acétylée | 6,7 % |
| - huile d'arara (esters d'acide oléique) | 13,5 % |
| - cire de lanoline oxypropylénée (5 OP) | 6,7 % |
| - érucate d'oléyle | 13,5 % |
| - triglycérides d'acides oleique-linoleique-linolenique | 1,7 % |
| - triglycérides d'acides palmitique-oleique-linoleique | 14,5 % |
| - tétraisopalmitate d'ascorbyle | 1,0 % |

### Exemple 6 :

Gloss apaisant

| | |
|---|---|
| - polybutène (Indopol H1500^{®} de Amoco) | 43,9 % |
| - benzoate d'alkyle en C₁₂-C₁₅ (FinsolvTN^{®} de Finetex) | 33,0 % |
| - copolymère PVP/hexadécène | 22,0 % |
| - octyldodécyl PCA (céramidone^{®} de Solabia) | 0,5 % |
| - α-hydroxypalmitoylsphingosine | 0,1 % |
| - éther cétylique de vitamine C (NIKKOL) | 0,5 % |

### Exemple 7 :

Rouge liquide pour lèvres fragiles

| | |
|---|---|
| - dispersion aqueuse de polyuréthanne (NEOREZ R-981) | qsp 100,00 % |
| - pigment | 1,00 % |
| - agent plastifiant (glycérine) | 1,25 % |
| - PCA de sodium | 1,00 % |
| - acide glycorpyranosyl-L-ascorbique (hayashibara) | 0,50 % |

### Exemple 8 :

Stick apaisant

| | |
|---|---|
| - hectorite modifiée par du chlorure de di-stearyl di-methyl Ammonium | 0,5% |
| - lanoline liquide | 26,2 % |
| - cire microcristalline | 10,5 % |
| - cire d'abeille polyglycérolée (3 moles) | 4,2 % |
| - lanoline acétylée | 7,7 % |
| - huile d'arara (esters d'acide oléique) | 13,5 % |
| - cire de lanoline oxypropylénée (5 op) | 6,7 % |
| - érucate d'oléyle | 13,5 % |
| - triglycérides d'acides palmitique-oleique-linoleique | 15,2 % |
| - lauryl PCA | 1,0 % |
| - tétraisopalmitate d'ascorbyle | 1,0 % |

## Revendications

1. Composition pour application labiale comprenant, dans un milieu physiologiquement acceptable, au moins la N-oléoyldihydrosphingosine et la α-hydroxypalmitoyldihydrosphingosine.

2. Composition pour application labiale selon la revendication 1, en association avec l'acide ascorbique ou au moins un de ses analogues choisis parmi ses sels, ses esters, ses sucres et les dérivés de formule (II) : 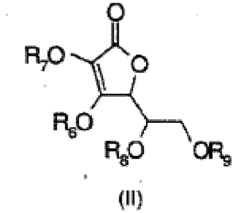 dans laquelle :
R₆ représente un atome d'hydrogène, une chaîne hydrocarbonée ayant de 15 à 20 atomes de carbone, un reste de sucre, un groupe carbonyle, un groupe alkyloxycarbonyle ou un groupe carbamoyle, ou bien R₆O représente une fonction sulfate ou une fonction phosphate,
R₇ représente un atome d'hydrogène, un reste de sucre ou un groupe -COR₁₀ où R₁₀ est choisi parmi :
(a) un radical hydrocarboné en C₁-C₂₀, saturé ou insaturé, linéaire, cyclique ou ramifié, éventuellement hydroxylé,
(b) un radical aryle éventuellement hydroxylé,
(c) un radical aralkyle de formule (III) : 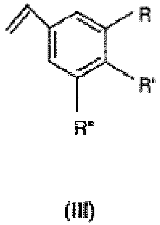
où R, R' et R", identiques ou différents, représentent un atome d'hydrogène, un radical hydroxy, alkoxy, fluoroalkoxy ou alkylcarbonyloxy, et
R₈, R₉, identiques ou différents, représentent un atome d'hydrogène ou un groupe-CORio tel que défini précédemment ou encore, pris ensemble, forment un radical isopropylidène, ou un sel de ce dérivé.

3. Composition pour application labiale selon la revendication précédente en association avec les analogues de l'acide ascorbique choisis parmi le palmitate d'ascorbyle, l'ascorbyl-2-phosphate de magnésium, l'acide glucopyranosyl-L-ascorbique, l'ascorbate de sodium, le L-ascorbyl-2-phosphate de sodium, le tetra-iso-palmitate d'ascorbyle et l'éther cétylique de vitamine C.

4. Composition pour application labiale selon l'une des revendications précédentes ladite composition se présentant sous forme de pâte, notamment de pâte souple, de solide, de crème, de mousse, de spray, de poudre ou de liquide.

5. Composition pour application labiale selon l'une des revendications précédentes, ladite composition se présentant sous forme d'une émulsion huile-dans-eau (H/E), huile-dans-polyol, eau-dans-huile (E/H), eau-dans-polyol, d'une émulsion multiple (E/H/E ou polyol/H/E ou H/E/H), d'un gel anhydre, d'un solide ou d'une pâte souple, d'une phase huileuse liquide, d'une suspension, d'une dispersion ou d'une solution.

6. Composition pour application labiale selon l'une des revendications précédentes, ladite composition se présentant sous forme de composition solide anhydre, et plus spécialement sous forme de stick.

7. Composition pour application labiale selon l'une des revendications précédentes, comprenant au moins la N-oléoyldihydrosphingosine et la α-hydroxypalmitoyldihydrosphingosine dans une teneur allant de 0,0001 à 10 % en poids, par exemple de 0,001 à 5 % en poids, et de préférence de 0,05 à 2% en poids par rapport au poids total de la composition.

8. Composition pour application labiale selon l'une quelconque des revendications 2 à 7, ladite composition comprenant de l'acide ascorbique et/ou au moins l'un de ses analogues dans une teneur totale allant de 0,0001 à 20 % en poids, par exemple de 0,001 à 10 % en poids, et de préférence de 0,01 à 10 % en poids par rapport au poids total de la composition.

9. Composition pour application labiale selon l'une quelconque des revendications précédentes, ladite composition se présentant sous la forme d'une composition dermatologique ou de soin des lèvres, sous la forme d'une composition de protection solaire des lèvres, sous la forme de base de soin pour les lèvres fragiles, sous la forme d'un baume à lèvres ou sous la forme d'un produit coloré de maquillage des lèvres, comme un rouge à lèvres ou un brillant à lèvres ou gloss.

10. Composition pour application labiale selon l'une quelconque des revendications précédentes, ladite composition étant épaissie avec au moins un agent épaississant choisi parmi les gélifiants de phase grasse, les cires, les corps gras pâteux, les charges et leurs mélanges.

11. Composition pour application labiale selon l'une quelconque des revendications précédentes, ladite composition comprenant au moins une matière colorante choisie parmi les colorants, les pigments, les nacres et leurs mélanges.

12. Composition pour application labiale selon l'une quelconque des revendications précédentes, ladite composition comprenant en outre au moins un actif additionnel choisi parmi les agents hydratants, les agents desquamants, les agents anti-pollution ou anti-radicalaire, les agents apaisants et les agents cicatrisants.

13. Composition pour application labiale selon la revendication précédente, l'agent hydratant étant choisi parmi les facteurs naturels d'hydratation dérivant de la dégradation de la filaggrine choisi parmi la sérine, le tryptophane, l'acide pyrrolidone carboxylique et l'acide urocanique, ainsi que leurs sels ou leurs dérivés.

14. Composition pour application labiale selon l'une quelconque des revendications précédentes, ladite composition comprenant en outre au moins un actif additionnel choisi parmi les extraits de Centella Asiatica, le madécassoside, la vitamine B5, l'hydroxyéthyl urée, l'oxyde de zinc et le C-β-D-xylopyranoside-2-hydroxy-propane.

15. Composition pour application labiale selon la revendication précédente, ladite composition comprenant en outre un ou plusieurs actifs additionnels dans une teneur totale allant de 0,001 à 30 % en poids, notamment de 0,01 à 20 % en poids par rapport au poids total de la composition.

16. Composition pour application labiale selon l'une quelconque des revendications précédentes pour son utilisation dans le traitement des signes localisés sur les vermillons des lèvres associés aux lèvres fragiles choisis parmi :
- la sensation d'inconfort et/ou de picotements ;
- la sensation de tiraillements ;
- la présence de squames ;
- les plaques sèches ;
- les fendillements ;
- les fissures ; et/ou
- les saignements,
la N-oléoyldihydrosphingosine et la α-hydroxypalmitoyldihydrosphingosine étant de conformation érythro (2S, 3R).

## Patentansprüche

1. Zusammensetzung zur labialen Anwendung, umfassend, in einem physiologisch annehmbaren Medium, mindestens N-Oleoyldihydrosphingosin und α-Hydroxypalmitoyldihydrosphingosin.

2. Zusammensetzung zur labialen Anwendung nach Anspruch 1, in Kombination mit Ascorbinsäure oder mindestens einem Analogon davon, ausgewählt aus Salzen davon, Estern davon, Zuckern davon und den Derivaten der Formel (II): 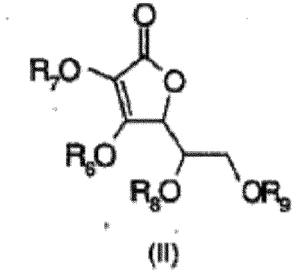 wobei:
R₆ ein Wasserstoffatom, eine Kohlenwasserstoffkette mit 15 bis 20 Kohlenstoffatomen, einen Zuckerrest, eine Carbonylgruppe, eine Alkyloxycarbonylgruppe oder eine Carbamoylgruppe bedeutet oder R₆O eine Sulfatfunktion oder eine Phosphatfunktion bedeutet, R₇ ein Wasserstoffatom, einen Zuckerrest oder eine -COR₁₀-Gruppe bedeutet, wobei R₁₀ aus Folgenden ausgewählt ist:
(a) einem gesättigten oder ungesättigten linearen, cyclischen oder verzweigten, gegebenenfalls hydroxylierten C₁-C₂₀-Kohlenwasserstoffrest,
(b) einem gegebenenfalls hydroxylierten Arylrest,
(c) einem Aralkylrest der Formel (III): 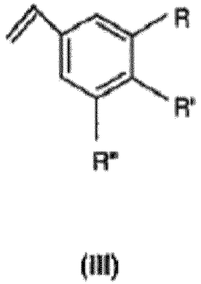
wobei R, R' und R", gleich oder unterschiedlich, ein Wasserstoffatom, einen Hydroxy-, Alkoxy-, Fluoralkoxy- oder Alkylcarbonyloxyrest bedeuten und
R₈, R₉, gleich oder unterschiedlich, ein Wasserstoffatom oder eine COR₁₀-Gruppe wie vorstehend definiert bedeuten oder zusammengenommen einen Isopropylidenrest oder ein Salz dieses Derivats bilden.

3. Zusammensetzung zur labialen Anwendung nach dem vorhergehenden Anspruch in Kombination mit den Analoga der Ascorbinsäure, ausgewählt aus Ascorbylpalmitat, Magnesium-Ascorbyl-2-Phosphat, Glucopyranosyl-L-Ascorbinsäure, Natriumascorbat, Natrium-L-Ascorbyl-2-phosphat, Ascorbyltetraisopalmitat und Vitamin-C-Cetylether.

4. Zusammensetzung zur labialen Anwendung nach einem der vorhergehenden Ansprüche, wobei die Zusammensetzung in Form einer Paste, insbesondere einer weichen Paste, eines Feststoffs, einer Creme, eines Schaums, eines Sprays, eines Pulvers oder einer Flüssigkeit vorliegt.

5. Zusammensetzung zur labialen Anwendung nach einem der vorhergehenden Ansprüche, wobei die Zusammensetzung in Form einer Öl-in-Wasser- (O/W), Öl-in-Polyol-, Wasser-in-Öl- (W/O), Wasser-in-Polyol-Emulsion, einer Mehrfachemulsion (W/O/W oder Polyol/O/W oder O/W/O), eines wasserfreien Gels, eines Feststoffs oder einer weichen Paste, einer flüssigen Ölphase, einer Suspension, einer Dispersion oder einer Lösung vorliegt.

6. Zusammensetzung zur labialen Anwendung nach einem der vorhergehenden Ansprüche, wobei die Zusammensetzung in Form einer festen wasserfreien Zusammensetzung, und insbesondere in Form eines Sticks vorliegt.

7. Zusammensetzung zur labialen Anwendung nach einem der vorhergehenden Ansprüche, umfassend mindestens N-Oleoyldihydrosphingosin und α-Hydroxypalmitoyldihydrosphingosin in einer Menge von 0,0001 bis 10 Gewichts-%, zum Beispiel von 0,001 bis 5 Gewichts-%, und bevorzugt von 0,05 bis 2 Gewichts-% bezogen auf das Gesamtgewicht der Zusammensetzung umfasst.

8. Zusammensetzung zur labialen Anwendung nach einem der Ansprüche 2 bis 7, wobei die Zusammensetzung Ascorbinsäure und/oder mindestens ein Analogon davon in einer Gesamtmenge von 0,0001 bis 20 Gewichts-%, zum Beispiel von 0,001 bis 10 Gewichts-%, und bevorzugt von 0,01 bis 10 Gewichts-% bezogen auf das Gesamtgewicht der Zusammensetzung umfasst.

9. Zusammensetzung zur labialen Anwendung nach einem der vorhergehenden Ansprüche, wobei die Zusammensetzung in Form einer dermatologischen oder Lippenpflegezusammensetzung, in Form einer Lippensonnenschutzzusammensetzung, in Form einer Pflegegrundlage für spröde Lippen, in Form eines Lippenbalsams oder in Form eines gefärbten Lippenschminkprodukts wie eines Lippenstifts oder eines Lipgloss oder Gloss vorliegt.

10. Zusammensetzung zur labialen Anwendung nach einem der vorhergehenden Ansprüche, wobei die Zusammensetzung mit mindestens einem Verdickungsmittel, ausgewählt aus Fettphasegeliermitteln, Wachsen, pastösen Fettsubstanzen, Füllstoffen und Gemischen davon verdickt wird.

11. Zusammensetzung zur labialen Anwendung nach einem der vorhergehenden Ansprüche, wobei die Zusammensetzung mindestens ein Färbemittel, ausgewählt aus Farbstoffen, Pigmenten, Perlglanzpigmenten und Gemischen davon umfasst.

12. Zusammensetzung zur labialen Anwendung nach einem der vorhergehenden Ansprüche, wobei die Zusammensetzung zudem mindestens einen zusätzlichen Wirkstoff, ausgewählt aus Feuchtigkeitsmitteln, abschuppenden Wirkstoffen, einem Antiverschmutzungs- oder Antiradikalmittel, beruhigenden Mitteln und Wundheilungsmitteln umfasst.

13. Zusammensetzung zur labialen Anwendung nach dem vorhergehenden Anspruch, wobei das Feuchtigkeitsmittel aus natürlichen Feuchthaltefaktoren, die aus dem Filaggrinabbau stammen, ausgewählt aus Serin, Tryptophan, Pyrrolidoncarbonsäure und Urocaninsäure sowie Salzen davon und Derivaten davon, ausgewählt ist.

14. Zusammensetzung zur labialen Anwendung nach einem der vorhergehenden Ansprüche, wobei die Zusammensetzung zudem mindestens einen zusätzlichen Wirkstoff, ausgewählt aus den Extrakten von Centella Asiatica, Madecassosid, Vitamin B5, Hydroxyethylharnstoff, Zinkoxid und C-β-D-Xylopyranosid-2-hydroxypropan, umfasst.

15. Zusammensetzung zur labialen Anwendung nach dem vorhergehenden Anspruch, wobei die Zusammensetzung zudem einen oder mehrere zusätzliche Wirkstoffe in einer Gesamtmenge von 0,001 bis 30 Gewichts-%, insbesondere 0,01 bis 20 Gewichts-% bezogen auf das Gesamtgewicht der Zusammensetzung umfasst.

16. Zusammensetzung zur labialen Anwendung nach einem der vorhergehenden Ansprüche zur Verwendung bei der Behandlung lokaler Erscheinungen auf dem Lippenrot im Zusammenhang mit spröden Lippen, ausgewählt aus:
- unangenehmem Gefühl und/oder Kribbeln;
- Spannungsgefühl;
- Vorhandensein von Schuppen;
- trockenen Stellen;
- Rissbildungen;
- Rissen; und/oder
- Bluten,
wobei N-Oleoyldihydrosphingosin und α-Hydroxypalmitoyldihydrosphingosin in Erythro-Konformation (2S, 3R) sind.

## Claims

1. A composition for labial application comprising, in a physiologically acceptable medium, at least N-oleoyldihydrosphingosine and α-hydroxypalmitoyldihydrosphingosine.

2. The composition for labial application according to claim 1, in association with ascorbic acid or at least one the analogues thereof selected from among the salts, esters, sugars thereof and derivatives of formula (II) : 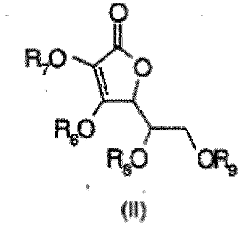 where:
R₆ is a hydrogen atom, a hydrocarbon chain having 15 to 20 carbon atoms, a sugar residue, a carbonyl group, an alkyloxycarbonyl group or carbamoyl group, or else R₆O is a sulfate function or phosphate function.
R₇ is a hydrogen atom, a sugar residue or -COR₁₀ group where
Rio is selected from among:
(a) a saturated or unsaturated, linear, cyclic or branched C₁-C₂₀ hydrocarbon radical, optionally hydroxylated,
(b) an aryl radical optionally hydroxylated,
(c) an aralkyl radical of formula (III): 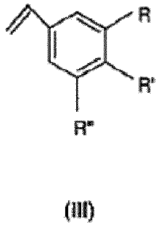
where R, R' and R", the same or different, are a hydrogen atom, a hydroxy, alkoxy, fluoroalkoxy or alkylcarbonyloxy radical, and
R₈, R₉, the same or different, are a hydrogen atom or - COR₁₀ group such as previously defined or, taken together, they form an isopropylidene radical or a salt of this derivative.

3. The composition for labial application according to the preceding claim, in association with analogues of ascorbic acid selected from among ascorbyl palmitate, magnesium ascorbyl-2-phosphate, glucopyranosyl-L-ascorbic acid, sodium ascorbate, sodium L-ascorbyl-2-phosphate, ascorbyl tetra-iso-palmitate and vitamin C cetyl ether.

4. The composition for labial application according to one of the preceding claims, said composition being in the form of a paste particularly a soft paste, a solid, cream, foam, spray, powder or liquid.

5. The composition for labial application according to one of the preceding claims, said composition being in the form of an oil-in-water (O/W), oil-in-polyol, water-in-oil (W/O) emulsion, a multiple emulsion (W/O/W or polyol/O/W or O/W/O), an anhydrous gel, a solid or soft paste, a liquid oily phase, a suspension, dispersion or solution.

6. The composition for labial application according to one of the preceding claims, said composition being in the form of an anhydrous solid composition and more especially in stick form.

7. The composition for labial application according to one of the preceding claims, comprising at least N-oleoyldihydrosphingosine and α-hydroxypalmitoyldihydrosphingosine in a content ranging from 0.0001 to 10 % by weight, for example 0.001 to 5 % by weight, preferably 0.05 to 2 % by weight relative to the total weight of the composition.

8. The composition for labial application according to any of claims 2 to 7, said composition comprising ascorbic acid and/or at least one of the analogues thereof in a total content ranging from 0.0001 to 20 % by weight, for example 0.001 to 10 % by weight, and preferably 0.01 to 10 % by weight relative to the total weight of the composition.

9. The composition for labial application according to any of the preceding claims, said composition being in the form of a dermatological or lip care composition, in the form of a lip sunscreen composition, in the form of a care base for sensitive lips, in the form of a lip balm or in the form of a coloured lip make-up product such as a lipstick or gloss.

10. The composition for labial application according to any of the preceding claims, said composition being thickened with at least one thickening agent selected from among fatty phase gelling agents, waxes, pasty fatty substances, fillers and mixtures thereof.

11. The composition for labial application according to any of the preceding claims, said composition comprising at least one colouring material selected from among colourants, pigments, pearling agents and mixtures thereof.

12. The composition for labial application according to any of the preceding claims, said composition further comprising at least one additional active substance selected from among moisturizing agents, peeling agents, anti-pollution or anti-radical agents, soothing agents and healing agents.

13. The composition for labial application according to the preceding claim, the moisturizing agent being selected from among natural moisturizing factors derived from filaggrin degradation selected from among serine, tryptophane, pyrrolidone carboxylic acid and urocanic acid, and the salts or derivatives thereof.

14. The composition for labial application according to any of the preceding claims, said composition further comprising at least one additional active substance selected from among extracts of *Centella Asiatica,* madecassoside, vitamin B5, hydroxyethyl urea, zinc oxide and C-β-D-xylopyranoside-2-hydroxy-propane.

15. The composition for labial application according to the preceding claim, said composition further comprising one or more additional active substances in a total content ranging from 0.001 to 30 % by weight, in particular 0.01 to 20 % by weight relative to the total weight of the composition.

16. The composition for labial application according to any of the preceding claims, for use thereof in the treatment of signs located on lip vermillion associated with sensitive lips selected from among:
- feelings of discomfort and/or stinging;
- feelings of tautness;
- presence of flaking;
- dry patches;
- chapping;
- cracking; and/or
- bleeding,
the N-oleoyldihydrosphingosine and a-hydroxypalmitoyldihydrosphingosine being of erythro conformation (2S, 3R).
